# EUROPEAN PATENT APPLICATION

(11) **EP 4 393 908 A1**
(43) Date of publication of application: **03.07.2024**
(21) Application number: 22383292.4
(22) Date of filing: 27.12.2022
(51) Int. Cl.: C07D 231/20, A61P 35/00, A61K 31/4155

(54) **BIS-PYRAZOLYL-METHANE COMPOUNDS AFFECTING KRAS**

(71) Applicant: Universitat de Barcelona, 08028 Barcelona (ES)
(72) Inventor: AGELL JANÉ, Nieves, 08036 Barelona (ES); PUJOL DILME, María, 08028 Barcelona (ES); RUBIO MARTÍNEZ, Jaime, 08028 Barcelona (ES); JAUMOT PIJOAN, Montserrat, 08036 Barcelona (ES); GARRIDO SAGARZAZU, Eduardo, 08028 Barcelona (ES); VILAPLANA SAIZ, Marta, 08028 Barcelona (ES); ABUASAKER, Baraa, 08036 Barcelona (ES); BRUN LOZANO, Sonia, 08036 Barcelona (ES)
(74) Representative: Manuel Illescas y Asociados, S.L.

(57) **Abstract**

The present invention relates to compounds of formula I 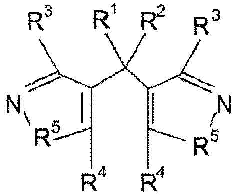 that can be used as medicaments for example in cancer therapy. The present invention further relates to pharmaceutical composition comprising the compounds as well as intermediates for the preparation of these compounds. In addition, the present invention concerns kits or packages comprising these compounds.

## Description

### Field of the invention

The present invention relates to compounds that can be used as medicaments for example in cancer therapy. The present invention further relates to pharmaceutical composition comprising the compounds as well as intermediates for the preparation of these compounds. In addition, the present invention concerns kits or packages comprising these compounds.

### Background of the invention

KRAS was the first oncogene identified in human cancer (Nobuo et al., 1982) and activating mutations affecting members of the RAS family genes (KRAS, HRAS, NRAS) are the most frequent genetic alterations, being found in about 27% of all tumors. KRAS is the most recurrently mutated gene among the Ras family (86% of RAS-mutant cancers) (Cox et al., 2014; Prior et al., 2020) and is a driver gene that is particularly involved in the development and progression of pancreatic (PDAC), lung and colorectal (CRC) cancers or other cancers (for recent reviews see (McCormick, 2019; Simanshu et al., 2017)). KRAS encodes for a small GTPase that in normal cells functions as a molecular switch cycling from a GDP-bound inactive to a GTP-bound active state (Karnoub and Weinberg, 2008; Malumbres and Barbacid, 2003). The main KRAS effectors are RAF, PI3K and Ral-GDS. Binding of RAF to KRAS induces unlocking of the closed conformation of RAF favoring its activation and consequently MEK and ERK activation. Targeting KRAS in cancer has been a central goal during the past four decades, but it is not until recently that all these efforts have started to bear fruit (McCormick, 2018, 2019). Sotorasib (AMG 510) and Adagrasib (MRTX849) are compounds that covalently bind to and block the oncogenic KRASG12C mutant and have been approved as second-line treatment for non-small cell lung cancer patients (NSCLC) with the KRASG12C- positive mutation (Canon et al., 2019; Hallin et al., 2020). Unfortunately, the frequency of this specific mutation is very low in PDAC and CRC and therefore other inhibitors need to be developed (Prior et al., 2020). Consequently, compounds that affect KRAS signaling are interesting therapeutics for different cancers.

### Brief description of the invention

A compound having the general formula I 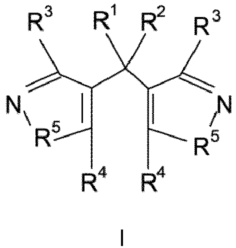 wherein
R¹ is selected from H, branched or linear (C₁-C₆)-alkyl, branched, cyclo or linear (C₁-C₆)-alkyl-OH, branched, cyclo or linear (C₁-C₆)-alkyl-NH₂, branched or linear (C₁-C₆)-alkyl comprising a secondary or tertiary amine or an ether group;
R² is selected from branched or linear (C₁-C₆)-alkyl, heterocyclyl, heteroaryl, substituted or unsubstituted (C₆-C₁₀)-aryl, or substituted or unsubstituted (C₅-C₈)-cycloalkenyl, wherein the substituted (C₆-C₁₀)-aryl or substituted (C₅-C₈)-cycloalkenyl is substituted with 1 or 2 groups selected from
   a) OCH₃,
   b) halogen,
   c) a substituent having formula II 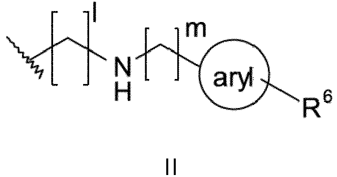 wherein "I" and "m" are independently selected from 0 to 6, the aryl group has 5 to 10 atoms, and wherein R⁶ is H, branched, cyclo or linear (C₁-C₆)-alkyl, or halogen;
   d) a substituent having formula III 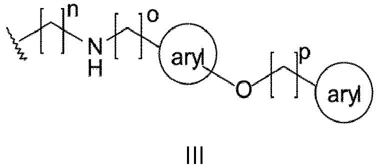 wherein the aryl groups independently have 5 to 10 atoms, and "n", "o" and "p" are independently selected from 0 to 6; and/or
   e) a substituent having formula IV 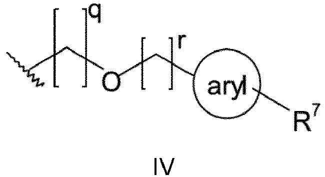 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1;
R₃ is independently selected from H, branched, cyclo or linear (C₁-C₆)-alkyl, branched, cyclo or linear (C₁-C₆)-alkyl-OH, CF₃, halogen, NO₂, and CN;
R₄ is independently selected from H, OH, O, NH₂, and NO₂; and
R⁵ is independently selected from N, NH, O, S, branched, cyclo or linear (C₁-C₆)-alkyl, branched or linear (C₁-C₆)-alkyl comprising a secondary or tertiary amine, an ether group or a sulfide group, N=N, and CH=N.

The present invention contemplates an intermediate compound in the synthesis of compounds of the present invention, wherein the intermediate compound is selected from
a) branched, cyclo or linear (C₁-C₆)-alkyl substituted with CHO;
b) heterocycle substituted with CHO;
c) (C₅-C₈)-cycloalkenyl substituted with CHO;
d) (C₆-C₁₀)-aryl substituted with CHO;
e) (C₅-C₈)-cycloalkenyl, or the (C₆-C₁₀)-aryl are substituted with CHO and with 1 or 2 groups selected from
   i) OCH₃;
   ii) halogen;
   iii) a substituent having formula II 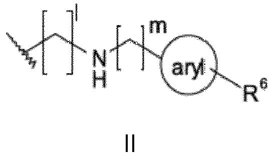 wherein "I" and "m" are independently selected from 0 to 6, the aryl group has 5 to 10 atoms, and wherein R⁶ is H, branched, cyclo or linear (C₁-C₆)-alkyl, or halogen;
   iv) a substituent having formula III 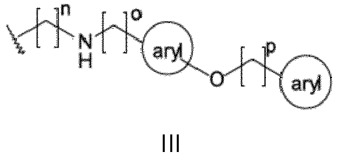 wherein the aryl groups independently have 5 to 10 atoms, and "n", "o" and "p" are independently selected from 0 to 6; and/or
   v) a substituent having formula IV 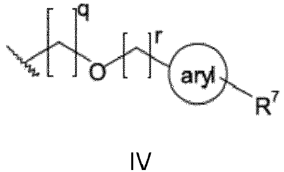 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.

The present invention also concerns the compound of the present invention or a salt or solvate thereof or a compound of formula 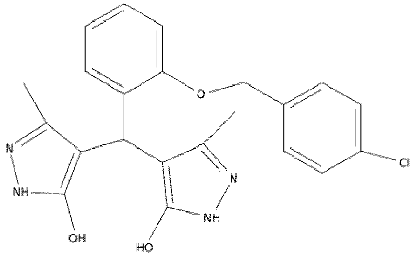 or a salt or solvate thereof for use as a medicament.

The present invention also relates to the compound of the present invention or a salt or solvate thereof or a compound of formula 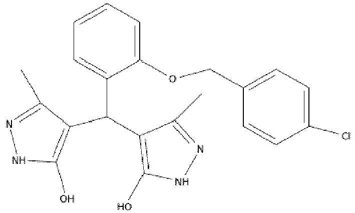 or a salt or solvate thereof for use in treating or preventing pancreatic cancer, lung cancer, colorectal cancer, thyroid cancer, testicle cancer, melanoma, bladder cancer, liver cancer, kidney cancer, myelodysplastic syndrome, or leukemia.

The present invention concerns a pharmaceutical composition comprising the compound of the present invention or a pharmaceutically acceptable salt or solvate thereof or a compound of formula 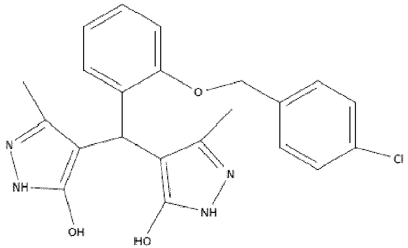 or salt or solvate thereof.

The present invention also relates to the pharmaceutical composition of the present invention for use as a medicament.

The present invention relates to the pharmaceutical composition of the present invention for use in treating pancreatic cancer, lung cancer, colorectal cancer, thyroid cancer, testicle cancer, melanoma, bladder cancer, liver cancer, kidney cancer, myelodysplastic syndrome, or leukemia.

The present invention concerns a method for the preparation of the present invention or the pharmaceutical composition of the present invention, the process comprising contacting 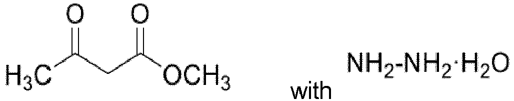 and with one intermediate compound of the present invention.

The present invention relates to a kit or package comprising the compound of the present invention or the pharmaceutical composition of the present invention.

### Brief description of the figures

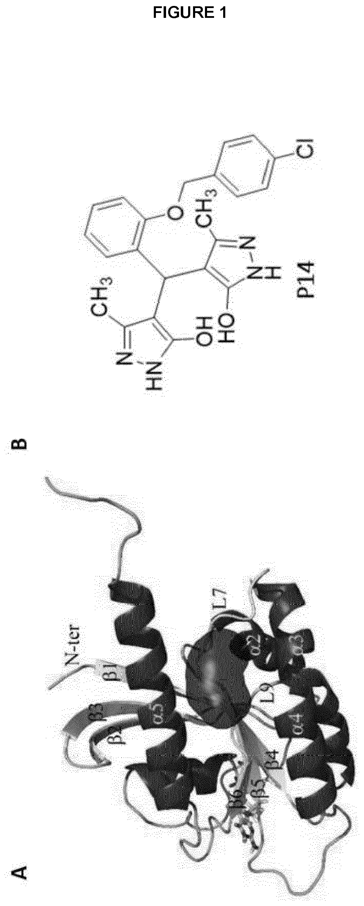

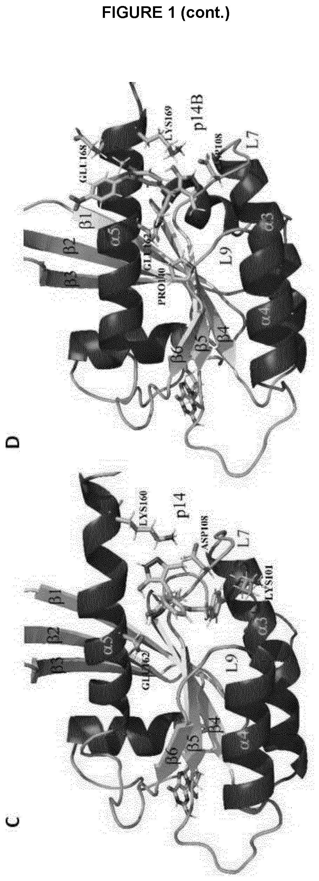

**Figure 1**. (A) KRAS binding site for calmodulin (CaM). Representation in blue surface of the binding site suggested both experimentally (Lopez-Alcalá et al., 2008) and theoretically (Garrido et al., 2018). (B) Formula of the compound P14. (C and D) Spatial representation of the KRAS-P14 and KRAS-P14B complex at the end of 200ns of conventional molecular dynamics.

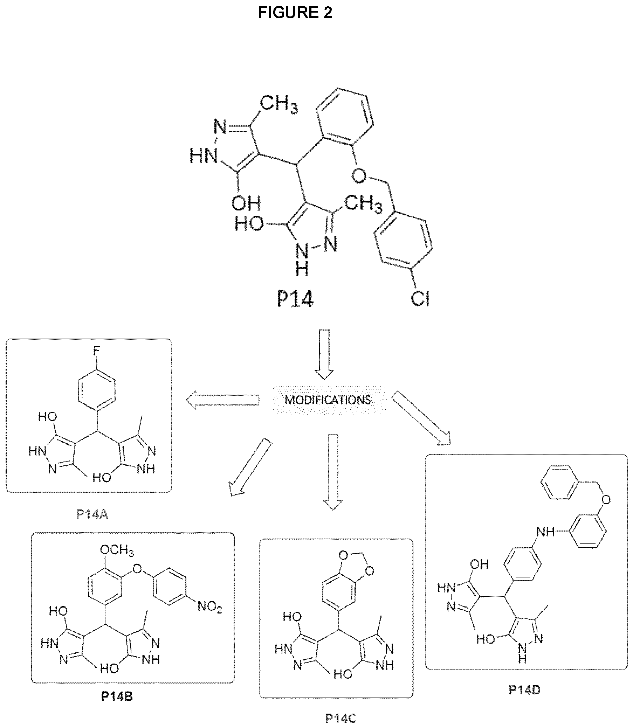

**Figure 2****.** Depiction of compounds of the present invention.

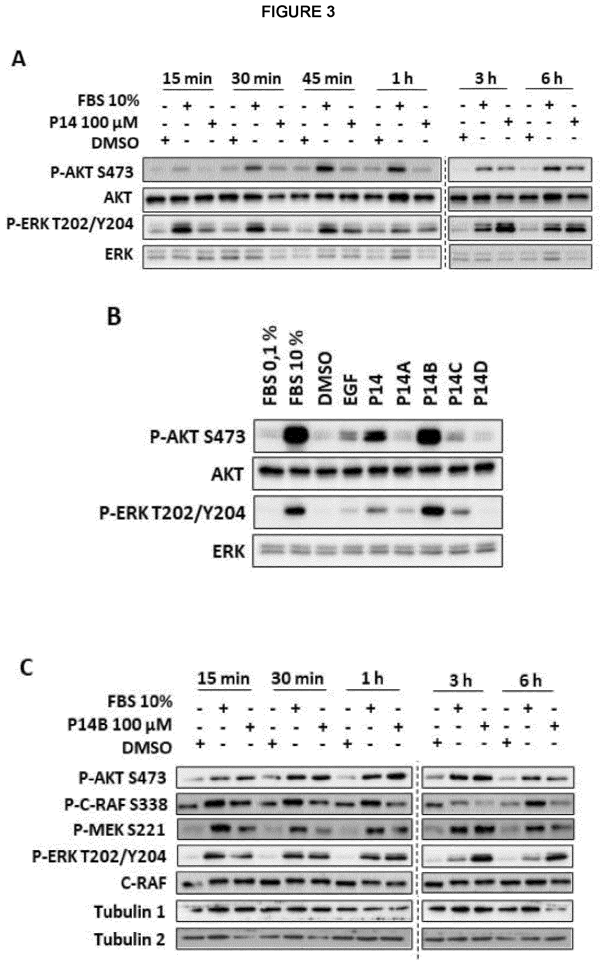

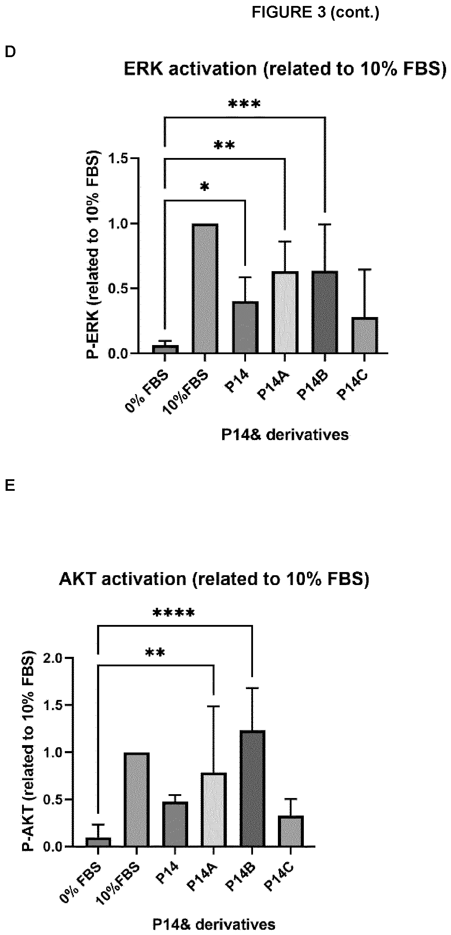

**Figure 3****.** Compounds of the invention induce higher activation of Ras effectors ERK and AKT than control. Treatment of DLD-1 (colorectal cancer cells with one oncogenic KRAS allele) cells increased endogenous downstream RAS signaling with different compounds of the invention. (A) Western blot analysis of DLD-1-starved cells incubated for different times with P14, with 10% FBS. (B) Western blot analysis of DLD-1 starved cells incubated for 3 hours, with P14B, P14C, or P14D or for 30 minutes with 10% FBS, or for 10 minutes with 50 ng/mL of EGF. (C). Western blot analysis of DLD-1 starved cells incubated for different times with either 10% FBS or P14B. In (A) and (B) the levels of activation and the total levels of ERK and AKT were analyzed by WB with specific antibodies against the active phosphorylated forms of these kinases or against the total forms of these proteins, respectively. In (C) activation of AKT, C-RAF. MEK and ERK was analysed using specific antibodies against the active phosphorylated forms and tubulin was used as loading control. Tubulin 1 corresponds to P-AKT and P-MEK gel, and Tubulin 2 to P-ERK, P-RAF and RAF gel. (D) and (E) show quantification of P14, P14A, P14B, or P14C of the Western blot shown in (B). Conclusion: All of P14, P14A, P14B, or P14C increase AKT and/or ERK signaling. P14A and P14B induce the highest activation of both Ras effectors: ERK and AKT.

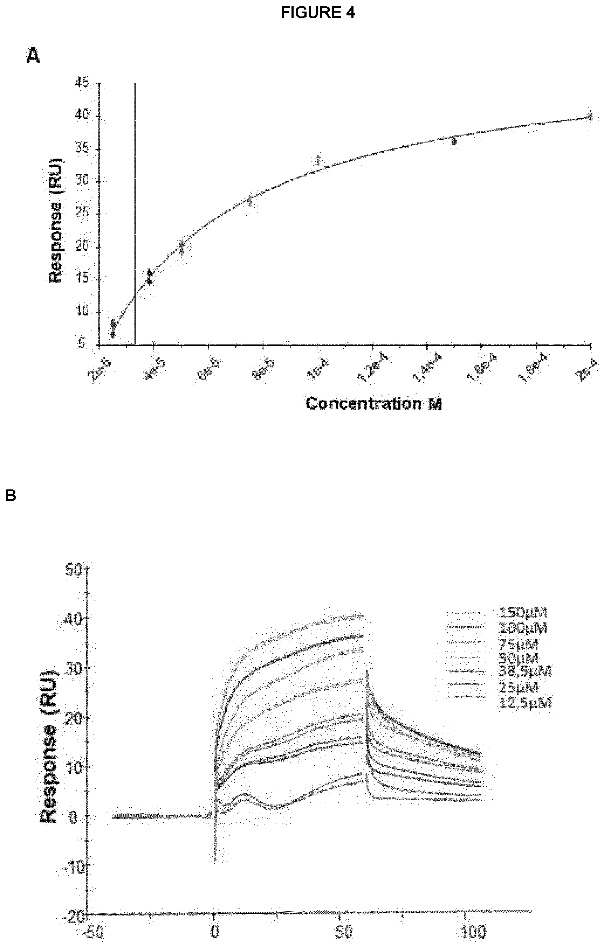

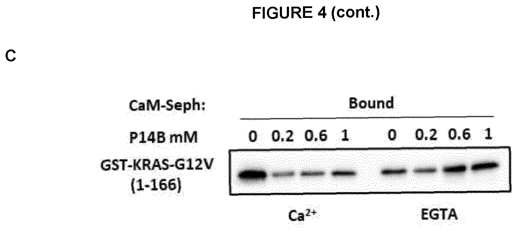

**Figure 4****.** (A, B) Kinetic analysis (A) and sensogrames (B) of the binding of P14B to GST-K-Ras-(1 166) determined by surface plasmon resonance. RU, resonance units. The same code of gray scale indicated in the kinetic analysis are used in the sensogram to indicate each concentration of P14B. (C) Competition between P14B and CaM for binding to oncogenic KRAS: CaM-sepharose pulldown assays were performed using GST-KRAS-G12V in the presence of P14B. Ca+2 or EGTA containing buffers indicate specific or non-specific KRAS-G12V binding to CaM, respectively. Bound fractions were analyzed by WB with anti-KRAS specific antibodies.

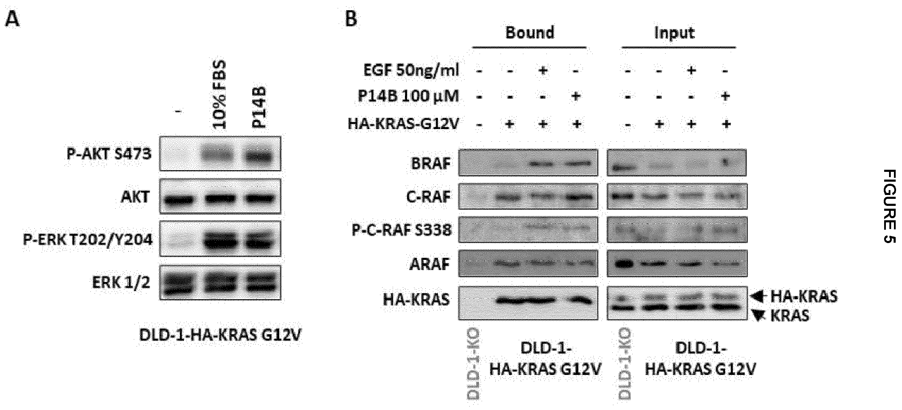

**Figure 5****.** P14B treatment increases oncogenic KRAS interaction with its effectors BRAF and C-RAF/P-C-RAF. (A) Serum starvedDLD-1 cells stably expressing HA-KRAS-G12V (DLD-1-HA-KRASG12V) were treated with 10%FBS (30 min) or P14B (3 h) and Western blots performed with the indicated antibodies. (B) Co-immunoprecipitation of HA-KRASG12V with BRAF, C-RAF/P-C-RAF, or ARAF was analyzed in starved DLD-1-KO cells stably expressing HA-KRASG12V after being treated with P14B (100µM) or EGF for 10 min. IP was performed with anti-HA antibodies and Western blot of the bound and input fractions with anti-BRAF, anti-C-RAF, anti-P-C-RAF(S338), and anti-ARAF-specific antibodies. (-) non-treated.

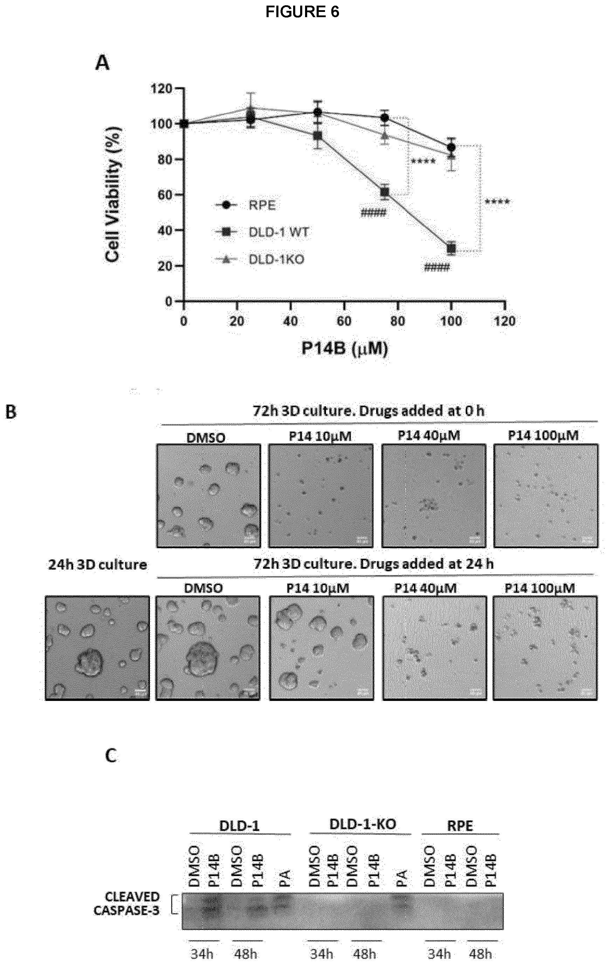

**Figure 6****.** P14B decreases the viability of CRC cells expressing oncogenic KRAS. (A) Effect of P14B on the viability of DLD-1, DLD-1-KO (DLD-1 cells with a deletion of the KRAS oncogenic allele) and hTERT-RPE cells. Cells were treated with P14B at a dose ranging from 0 to 100 at which time cell viability was determined by MTS assay. The experiment was repeated at least three times. Differences were assessed using oneway ANOVA and Multiple Comparisons Test. **** means p<0.0001 between DLD-1 and RPE or DLD-1-KO cells; #### means p<0.0001 between different concentration of P14B treated and non-treated DLD-1 cells.. (B) In total 2.5x104 DLD-1 cells were cultured on top of a thin basement membrane matrix (Matrigel) overlaid with a dilute solution of this basement membrane matrix (3D on-top Matrigel assay). At the time of seeding or 24h later, cells were treated with P14B (10 3 days after seeding the colonies that had formed were analysed. Representative phase-contrast images are shown. All scale bars, 40 (C) DLD-1 cells treated with P14B for 36 h and 48 h were lysed and the expression of the apoptosis marker cleaved caspase-3 was analysed by WB. Cells treated with the apoptosis inducer palmitic acid (PA) were used as a positive control.

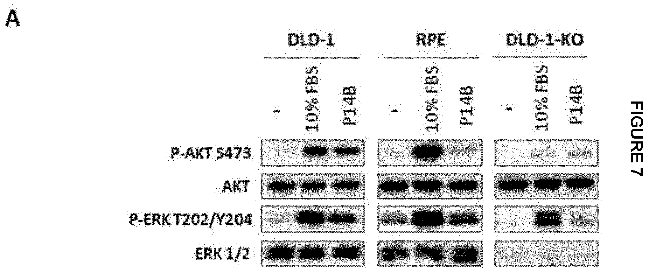

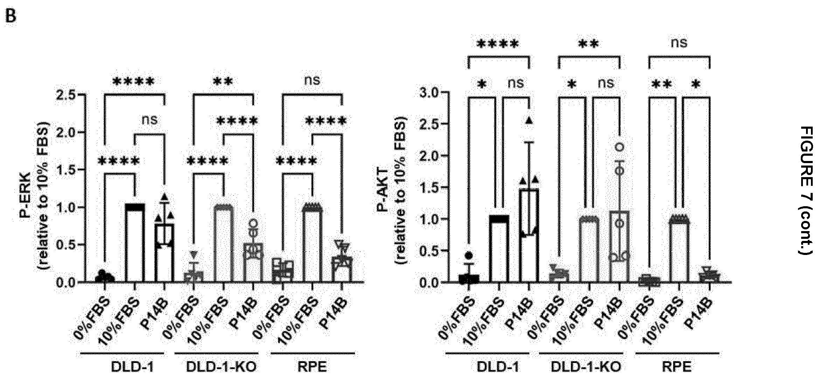

Figure 7. P14B treatment of CRC cells expressing oncogenic KRAS increases endogenous downstream RAS signaling. (A) DLD-1, DLD-1-KO and hTERT-RPE serum -starved cells were incubated with 10% FBS or P14B and activation of ERK and AKT were analyzed by WB. Specific antibodies against the active phosphorylated and total proteins were used. (B) Graph showing quantification of four independent experiments. Differences were assessed using oneway ANOVA and Multiple C -value < 0.05; **: p-value < 0.01; ****: p-value < 0.0001; and, ns: non-significant.

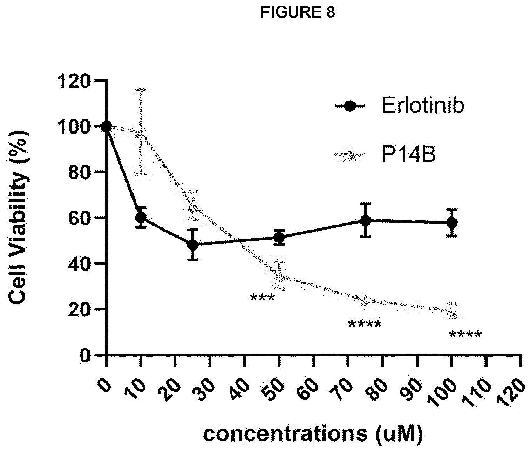

**Figure 8****.** Effect of Erlotinib and P14B on cell viability of DLD-1 cell line treated with different concentrations of the indicated compounds. In DLD1 cells, the maxim cell viability reduction induced by P14B is significantly higher than the one induced by Erlotinib. Differences were assessed using t-studemt ***: p-value < 0.001; ****: p-value < 0.0001.

### Detailed description of the invention

The present invention relates to a compound having the general formula I 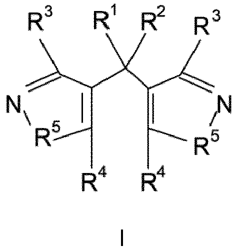 wherein
R¹ is selected from H, branched, cyclo or linear (C₁-C₆)-alkyl, branched, cyclo or linear (C₁-C₆)-alkyl-OH, branched, cyclo or linear (C₁-C₆)-alkyl-NH₂, optionally substituted with one or more halogens, branched or linear (C₁-C₆)-alkyl comprising a secondary or tertiary amine or an ether group;
R² is selected from branched, cyclo or linear (C₁-C₆)-alkyl, heterocyclyl, heteroaryl, substituted or unsubstituted (C₆-C₁₀)-aryl, or substituted or unsubstituted (C₅-C₈)-cycloalkenyl,
wherein the substituted (C₆-C₁₀)-aryl or substituted (C₅-C₈)-cycloalkenyl is substituted with 1 or 2 groups selected from the groups consisting of
   a) OCH₃,
   b) halogen,
   c) a substituent having formula II 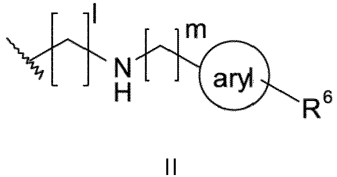 wherein "I" and "m" are independently selected from 0 to 6, the aryl group has 5 to 10 atoms, and wherein R⁶ is H, branched, cyclo or linear (C₁-C₆)-alkyl, or halogen;
   d) a substituent having formula III 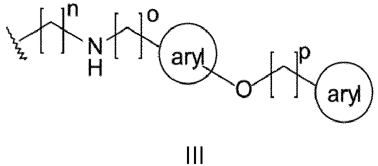 wherein the aryl groups independently have 5 to 10 atoms, and "n", "o" and "p" are independently selected from 0 to 6; and/or
   e) a substituent having formula IV 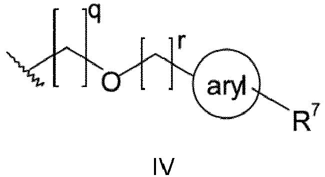 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1;
R₃ is independently selected from H, branched, cyclo or linear (C₁-C₆)-alkyl, branched, cyclo or linear (C₁-C₆)-alkyl-OH, CF₃, halogen, NO₂, and CN;
R₄ is independently selected from H, OH, O, NH₂, and NO₂; and
R⁵ is independently selected from N, NH, O, S, branched, cyclo or linear (C₁-C₆)-alkyl, branched or linear (C₁-C₆)-alkyl comprising a secondary or tertiary amine, an ether group or a sulfide group, N=N, and CH=N.

Thus, R¹ can be selected from H, branched, cyclo or linear (C₁-C₆)-alkyl, branched, cyclo or linear (C₁-C₆)-alkyl-OH, branched, cyclo or linear (C₁-C₆)-alkyl-NH₂, optionally substituted with one or more halogens, branched or linear (C₁-C₆)-alkyl comprising a secondary or tertiary amine or an ether group.

The term "alkyl" as used herein is defined as a saturated monovalent hydrocarbon moiety having straight or branched moieties or combinations thereof and containing 1 to 6 carbon atoms, preferably 1 to 3 carbon atoms. Alkyl moieties can optionally be substituted with cycloalkyl groups, -OH groups, NH₂ groups, and/or halogen. They can also be substituted by an ester group, a sulfide group or secondary or tertiary amine group. The term alkyl includes branched, linear and cycloalkyls.

The term "cycloalkyl" as used herein refers to a monovalent or divalent group of 3 to 8 carbon atoms, preferably 5 to 6 carbon atoms of saturated cyclic hydrocarbon. Cycloalkyl groups may optionally be substituted by alkyl groups, -OH groups, -NH₂ groups, and/or halogen.

As used herein the linear, cyclo or branched (C₁-C₆)-alkyl can be any branched, cyclo or linear C₁-, C₂-, C₃-, C₄, C₅ or C₆ alkyl. For example, C₁-alkyl can be methyl. C₂-alkyl can be ethyl.C₃-alkyl can be selected from the group consisting of propyl and cyclopropyl, preferably propyl. C₄-alkyl can be selected from the group consisting of n-butyl, isopropyl, butan-2-yl, 2-methylpropyl, tert-butyl, cyclobutyl and methylcyclopropyl, preferably n-butyl, isopropyl, butan-2-yl, 2-methylpropyl, tert-butyl, most preferably n-butyl. C₅-alkyl can be selected from the group consisting of n-pentyl, 2-methylbutyl, 2,2-dimethylpropyl, 3-methylbutyl, pentan-2-yl, pentan-3-yl, 3-methylbutan-2-yl, 2-methylbutyl, cyclopentyl, methylcyclobutyl, 1,1-dimethylcyclopropyl and 1,2-dimethylcyclopropyl, preferably n-pentyl, 2-methylbutyl, 2,2-dimethylpropyl, 3-methylbutyl, pentan-2-yl, pentan-3-yl, 3-methylbutan-2-yl, 2-methylbutyl, most preferably n-pentyl. C₆-alkyl can be selected from the group consisting of n-hexyl, 2-methylpentyl, 3-methylpentyl, 2,2-dimethylbutyl, 2,3-dimethylbutyl, cyclohexyl, methylcyclopentyl, 1,2-dimethylcyclobutyl, 1,3-dimethylcyclobutyl and 1,2,3-trimethylcyclopropyl, preferably n-hexyl, 2-methylpentyl, 3-methylpentyl, 2,2-dimethylbutyl, 2,3-dimethylbutyl, most preferably n-hexyl.

As used herein the linear or branched (C₁-C₆)-alkyl can be any branched or linear C₁-, C₂-, C₃-, C₄, C₅ or C₆ alkyl. For example, C₁-alkyl can be methyl. C₂-alkyl can be ethyl. C₃-alkyl can be propyl. C₄-alkyl can be selected from the group consisting of n-butyl, isopropyl, butan-2-yl, 2-methylpropyl, tert-butyl, preferably n-butyl. C₅-alkyl can be selected from the group consisting of n-pentyl, 2-methylbutyl, 2,2-dimethylpropyl, 3-methylbutyl, pentan-2-yl, pentan-3-yl, 3-methylbutan-2-yl, 2-methylbutyl, preferably n-pentyl. C₆-alkyl can be selected from the group consisting of n-hexyl, 2-methylpentyl, 3-methylpentyl, 2,2-dimethylbutyl, 2,3-dimethylbutyl, preferably n-hexyl.

Branched, cyclo or linear (C₁-C₆)-alkyl-OH as used herein means any branched, cyclo or linear (C₁-C₆)-alkyl-OH. The OH group may be attached to C₁, C₂, C₃, C₄, C₅ or C₆. It is envisioned that the OH group is attached to C₆. (C₁)-alkyl-OH can be methyl-OH. (C₂)-alkyl-OH may be ethyl-OH. (C₃)-alkyl-OH can be selected from the group consisting of propyl-OH and cyclopropyl-OH. (C₄)-alkyl-OH can be selected from the group consisting of n-butyl-OH, isopropyl-OH, butan-2-yl-OH, 2-methylpropyl-OH, tert-butyl-OH, cyclobutyl-OH and methylcyclopropyl-OH. (C₅)-alkyl-OH can be selected from the group consisting of n-pentyl-OH, 2-methylbutyl-OH, 2,2-dimethylpropyl-OH, 3-methylbutyl-OH, pentan-2-yl-OH, pentan-3-yl-OH, 3-methylbutan-2-yl-OH, 2-methylbutyl-OH, cyclopentyl-OH, methylcyclobutyl-OH, 1,1-dimethylcyclopropyl-OH and 1,2-dimethylcyclopropyl-OH. (C₆)-alkyl-OH can be selected from the group consisting of n-hexyl-OH, 2-methylpentyl-OH, 3-methylpentyl-OH, 2,2-dimethylbutyl-OH, 2,3-dimethylbutyl-OH, cyclohexyl-OH, methylcyclopentyl-OH, 1,2-dimethylcyclobutyl-OH, 1,3-dimethylcyclobutyl-OH and 1,2,3-trimethylcyclopropyl-OH.

Branched, cyclo or linear (C₁-C₆)-alkyl-NH₂ as used herein means any branched, cyclo or linear (C₁-C₆)-alkyl-NH₂ The NH₂ group may be attached to C₁, C₂, C₃, C₄, C₅ or C₆. It is envisioned that the NH₂ group is attached to C₆. (C₁)-alkyl-NH₂ can be methyl-NH₂. (C₂)-alkyl- NH₂ can be ethyl-NH₂. (C₃)-alkyl- NH₂ can be selected from the group consisting of propyl-NH₂ and cyclopropyl-NH₂ (C₄)-alkyl- NH₂ can be selected from the group consisting of *n*-butyl-NH₂, isopropyl-NH₂, butan-2-yl-NH₂, 2-methylpropyl-NH₂, tert-butyl- NH₂, cyclobutyl-NH₂ and methylcyclopropyl-NH₂. (C₅)-alkyl-NH₂ can be selected from the group consisting of *n*-pentyl-NH₂, 2-methylbutyl-NH₂, 2,2-dimethylpropyl-NH₂, 3-methylbutyl-NH₂, pentan-2-yl-NH₂, pentan-3-yl-NH₂, 3-methylbutan-2-yl-NH₂, 2-methylbutyl-NH₂, cyclopentyl-NH₂, methylcyclobutyl-NH₂, 1,1-dimethylcyclopropyl-NH₂ and 1,2-dimethylcyclopropyl-NH₂. (C₆)-alkyl-NH₂ can be selected from the group consisting of *n*-hexyl-NH₂, 2-methylpentyl-NH₂, 3-methylpentyl-NH₂, 2,2-dimethylbutyl-NH₂, 2,3-dimethylbutyl-NH₂, cyclohexyl-NH₂, methylcyclopentyl-NH₂, 1,2-dimethylcyclobutyl-NH₂, 1,3-dimethylcyclobutyl-NH₂ and 1,2,3-trimethylcyclopropyl-NH₂.

It is also contemplated by the present invention that the branched, cyclo or linear (C₁-C₆)-alkyl-NH₂ is further substituted with one or more halogens. The NH₂ group and/or the halogen may be attached to C₁, C₂, C₃, C₄, C₅ or C₆. As such the halogen with which the (C₁-C₆)-alkyl-NH₂ is further substituted may be CF₃. The branched, cyclo or linear (C₁-C₆)-alkyl-NH₂ can thus be substituted with one, two, three or four or more halogens, preferably CF₃. Therefore, the (C₁-C₆)-alkyl-NH₂ that is further substituted with a halogen may be a trifluoromethylcypropyl-NH₂.

The branched or linear (C₁-C₆)-alkyl comprising a secondary or tertiary amine or an ether group as used herein refers to any branched or linear (C₁-C₆)-alkyl comprising a secondary or tertiary amine or an ether group.

The skilled person knows that the branched or linear (C₁-C₆)-alkyl comprising a secondary amine has a formula of (C₁-C₅)-alkyl-NH-(C₁-C₅)-alkyl, or pyrrole/isothiazole/isoxazole/pyrazole of formula I-NH-(C₁-C₆)-alkyl. This means that the N atom has three substituents. Two substituents are, independently, branched or linear (C₁-C₅)-alkyl as defined elsewhere herein, and one is H. It is also contemplated that the NH group is attached to the pyrrole/isothiazole/isoxazole/pyrazole of formula I of formula I and further comprises a branched or linear (C₁-C₆)-alkyl chain and a H as further substituents.

The skilled person understands that the branched or linear (C₁-C₆)-alkyl comprising a tertiary amine has a formula of (C₁-C₄)-alkyl-N(C₁-C₄)-alkyl-(C₁-C₄)-alkyl or pyrrole/isothiazole/isoxazole/pyrazole of formula I- N(C₁-C₄)-alkyl-(C₁-C₄)-alkyl. This means that the N atom has three substituents all of which can independently be branched or linear (C₁-C₄)-alkyl as defined elsewhere herein. It is also envisioned that the N atom has three substituents of which two can independently be branched or linear (C₁-C₅)-alkyl as defined elsewhere herein and the other substituent is the pyrrole/isothiazole/isoxazole/pyrazole of formula I.

Further, one skilled in the art knows that a branched or linear (C₁-C₆)-alkyl comprising an ether refers to the formula (C₁-C₅)-alkyl-O-(C₁-C₅)-alkyl or pyrrole/isothiazole/isoxazole/pyrazole of formula I-O-(C₁-C₆)-alkyl. This means that the O atom has two substituents. Both substituents can independently be branched or linear (C₁-C₅)-alkyl as defined elsewhere herein. It is further envisioned that one substituent is the pyrrole/isothiazole/isoxazole/pyrazole of formula I and the other one a (C₁-C₆)-alkyl as elsewhere defined herein.

The secondary or tertiary amine or the ether group can thus be positioned before C₁, between C₁ and C₂, C₂ and C₃, C₃ and C₄, C₄ and C₅ or C₅ and C₆ or on C₆. Preferably, the secondary or tertiary amine or the ether is located on C₆ or on the last carbon atom of the alkyl group if the alkyl group has less than 6 carbon atoms. Additionally, or alternatively, the secondary or tertiary amine or the ether is located before C₁ or between C₁ and C₂. The term before C₁ indicates that the NH, N or O is located between the pyrrole/isothiazole/isoxazole/pyrazole of formula I and the further substituents as described elsewhere herein e.g. one or two (C₁-C₆)alkyl chain.

In these scenarios, the tertiary amine can, for example, additionally be attached to a (C₁-C₆)-alkyl group. Preferably, the alkyl group is a methyl, ethyl or propyl group, most preferably a CH₃ group. That means that the tertiary amine is e.g., located before C₁ and additionally includes a CH₃ group.

It is thus further contemplated that the secondary or tertiary amine or an ether group comprised in the branched or linear (C₁-C₆)-alkyl is located before C₁, between C₁ and C₂ or on C₆.

(C₂)-alkyl comprising a secondary amine or an ether group can be methyl-O-methyl or methyl-NH-methyl. (C₃)-alkyl comprising a secondary amine or an ether group can be selected from methyl-O-ethyl, ethyl-O-methyl, methyl-NH-ethyl or ethyl-NH-methyl. (C₄)-alkyl comprising a secondary amine or an ether group can be selected from methyl-O-propyl, ethyl-O-ethyl, propyl-O-methyl, methyl-NH-propyl, ethyl-NH-ethyl, propyl-NH-methyl. (C₅)- alkyl comprising a secondary amine or an ether group can be selected from methyl-O-butyl, propyl-O-ethyl, ethyl-O-propyl, butyl-O-methyl, methyl-NH-butyl, propyl-NH-ethyl, ethyl-NH-propyl, butyl-NH-methyl. (C₆)- alkyl comprising a secondary amine or an ether group can be selected from methyl-O-pentyl, butyl-O-ethyl, propyl-O-propyl, ethyl-O-butyl, pentyl-O-methyl, methyl-NH-pentyl, butyl-NH-ethyl, propyl-NH-propyl, ethyl-NH-butyl, pentyl-NH-methyl.

R¹ can be, for example, be selected from H, methyl, ethyl, n-propyl, n-butyl, n-pentyl, *n-*hexyl, methyl-OH, ethyl-OH, propyl-OH, n-butyl-OH, n-pentyl-OH, n-hexyl-OH, methyl-NH₂, ethyl-NH₂, propyl-NH₂ *n*-butyl-NH₂, *n*-pentyl-NH₂, *n*-hexyl-NH₂, trifluoromethylcypropyl-NH₂,

It is envisioned that R¹ is selected from H and CH₃. It is also contemplated that R¹ is H. R² can be selected from branched, cyclo or linear (C₁-C₆)-alkyl, heterocyclyl, heteroaryl, substituted or unsubstituted (C₆-C₁₀)-aryl, or substituted or unsubstituted (C₅-C₈)-cycloalkenyl,

Heterocyclyl as used herein refers to any saturated or partially unsaturated cyclic alkyl group with one or more ring heteroatoms independently selected from nitrogen, oxygen and sulfur. The term heterocyclyl includes heteroalkenyl groups (i.e. the heterocyclyl group having at least one double bond), bicyclic heterocyclic groups such as bridged-heterocyclyl groups or fused heterocyclyl groups. Thus, a heterocyclyl may be a single ring or multiple rings wherein the multiple rings may be fused or bridged and may comprise one or more (e.g. 1 to 3) oxygen (O), nitrogen (N) and/or sulfur (S) groups. The term heterocyclyl as used herein excludes heteroaryls. Exemplary heterocyclyls can thus be pyrrolidine, 3-pyrroline, 2-pyrroline, pyrazolidine, imidazolidine, 2-pyrazoline, 2-imidazoline, tetrahydrofuran, 1,3-dioxolane, tetrahydrothiophene, 1,2-oxathiolane, 1,3-oxathiolane, piperidine, piperazine, tetrahydropyran, 2*H*-pyran, 4*H*-pyran, 1,4-dioxane, 1,4-dioxine, 1,3- or 1,4-thiane, 1,3,5-trithiane, morpholine, 4*H*-1,2-oxazine, 2H-1,2-oxazine, 6*H*-1,2-oxazine, 4*H*-1,3-oxazine, 2*H*-1,3-oxazine, 6H-1,3-oxazine, 4H-1,4-oxazine, 2*H*-1,4-oxazine, thiomorpholine, 4H-1,4-thiazine, 2*H*-1,4-thiazine, 6H-1,2-thiazine, 2H-1,4-thiazine, decahydroisoquinoline, decahydroquinoline and the like.

The heteroaryl as used herein refers to any heteroaryl. Heteroaryl as used herein refers to an aromatic group having a single ring, multiple bridged rings or multiple fused rings with one or more ting heteroatoms independently selected from nitrogen, oxygen and sulfur. A heteroaryl can include 5 to 12 atoms, of which 1 to 4 are ring heteroatoms, wherein the heteroatom is independently nitrogen, oxygen and/or sulfur and 1 to 11 or 0 to 8 are carbon atoms. As used herein heteroaryl can also include 5 to 10 atoms, of which 1 to 4 are ring heteroatoms, wherein the heteroatom is independently nitrogen, oxygen and/or sulfur and 3 to 8 or 1 to 6 are carbon atoms. It is also envisioned that the heteroaryl can include 5 to 10 atoms, of which 1 to 3 are ring heteroatoms, wherein the heteroatom is independently nitrogen, oxygen and/or sulfur and 2 to 7 or 2 to 5 are carbon atoms. Any aromatic ring having a single or multiple fused rings, containing at least one heteroatom is considered a heteroaryl, regardless of the attachment to the remainder of the molecule. The term heteroaryl is also embraced by the term aryl as defined herein. Heteroaryls are known to the skilled person.

Exemplary heteroaryls include 2H-pyrrole, 1H-pyrrole, pyrazole, imidazole, 1,2,4-triazole, 1,2,3-triazole, tetrazole, furan, thiophene, oxazole, isoxazole, isothiazole, thiazole, -, 2, 5-oxadiazole, 1,3,4-thiadiazole, 1,2,5-thiadiazole, pyridine, pyridiazine, pyrimidine, pyrazine, 1,2,4-triazine, 1,3,5-triazine, 1,4,5,6-tetrahydrocyclopental[b]pyrrole, 1,4-dihydropyrrolo[3,2-b]pyrrole, 1,4-dihydropyrrolo[3,2-b]pyrrole, 6*H*-furo[2,3-*b*]pyrrole, 4*H*-furo[3,2-*b*]pyrrole, 6H-thieno [3,2-b]pyrrole, 6*H*-thieno[2,3-*b*]pyrrole, indene, 2,3-dihydro-1/7-indene, indoline, 3*H-*indole, 1H-indole, 2*H*-isoindole, indolizine, 1H-indazole, benzimidazole, 7-azaindole, 4-azaindole, 5-azaindole, 6-azaindole, 7-azaindole, pyrazolo[1,5-a]pyrimidine, purine, benzofuran, isobenzofuran, benzo[c]thiophene, benzo[b]thiophene, 1,2-benzisoxazole, anthranil, 1,2-benzisothiazole, benzoxazole, benzthiazole, quinoline, quinoxaline, phthalazine, 2H-chromene and the like. Examples of five membered monocyclic heteroaryl groups include but are not limited to pyrrole, furan, thiophene, imidazole, furazan, oxazole, oxadiazole, oxatriazole, isoxazole, thiazole, isothiazole, pyrazole, triazole and tetrazole groups.

It is further contemplated that the heteroaryl can be selected from indolyl, pyrrolyl, furanyl, thiophenyl, pyridyl, pyrimidyl, pyridazyl, pyrazinyl, quinolinyl, isoquinolinyl, acridinyl, 1,2-methylenedioxyphenyl (= benzo-1,4-dioxanyl) or 1,2-ethylenedioxyphenyl (= 1,3-Benzodioxolyl), preferably 1-*H*-indolyl, 1,2-methylenedioxyphenyl and 1,2-ethylenedioxyphenyl.

As used herein (C₅-C₈)-cycloalkenyl refers to a cycloalkyl group containing at least one carbon-carbon double bond and having 5 to 8 carbon atoms.

As used herein the substituted or unsubstituted (C₅-C₈)-cycloalkenyl can be any substituted or unsubstituted (C₅-C₈)-cycloalkenyl. The substituent(s) may be attached to C₅ C₆, C₇, and/or C₈. For example, the substituted or unsubstituted (C₅-C₈)-cycloalkenyl can be selected from cyclopentenyl, cyclopentadienyl, cyclohexenyl, 1,3-cyclohexadiene, cycloheptenyl, 1,3-cycloheptadienyl, cycloheptatrienyl, cyclooctenyl, 1,5-cyclooctadienyl, or cyclooctatetraenyl.

Aryl as used herein refers to an aromatic carbocyclic group having a single ring (i.e. monocyclic) or multiple rings (e.g. bicyclic or tricyclic) including fused systems. As used herein an aryl group can have 6 to 10 ring atoms. These atoms can be carbon atoms in the case of aryls and carbon atoms and heteroatoms in the case of heteroaryls.

The term aryl includes aryls only comprising carbon atoms as well as heteroaryls as defined elsewhere herein.

The substituted or unsubstituted (C₆-C₁₀)-aryl can be any substituted or unsubstituted (C₆-C₁₀)-aryl. The substituent(s) may be attached to C₆, C₇, C₈, C₉ and/or C¹⁰. Examples include substituted or unsubstituted phenyl, substituted or unsubstituted tolyl, substituted or unsubstituted xylyl, and substituted or unsubstituted naphthyl, preferably substituted phenyl.

As used herein the term substituted means that one or more (e.g. 1 to 5 or 1 to 3) hydrogen atoms of the group is replaced with a substituent atom or group. Each substituent can be the same or different. Suitable substituents are described herein.

It is contemplated that R² can be indol-2- or 3-yl, pyrrol-3-yl, substituted phenyl, 1,2-methylenedioxyphenyl, 1,2-ethylenedioxyphenyl, 2- or 3-pyrrolyl, 2- or 3-furanyl, 2- or-thiophenyl, 2-, 3- or 4-pyridyl, 2- 4 or 6-pyrimidyl, quinolinyl, isoquinolinyl.

The aryl group having 5 to 10 atoms in formulae (II), (III) and/or (IV) can be any aryl group having 5 to 10 atoms in formula (II), (III) and/or (IV). The aryl group includes aryl groups having only carbon atoms as well as heteroaryl groups as described elsewhere herein. Examples include phenyl, pyrrolyl, furanyl, thiophenyl, pyridyl, pyrimidyl, quinolinyl and/or isoquinolinyl, preferably phenyl.

The present invention envisioned that the (C₆-C₁₀)-aryl or substituted (C₅-C₈)-cycloalkenyl can be substituted. For example, they can be substituted with OCH₃ and a substituent having formula IV 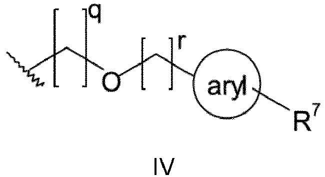 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.

It is further envisioned that the substituted (C₆-C₁₀)-aryl or substituted (C₅-C₈)-cycloalkenyl can be substituted at positions *ortho, meta* and/or *para* or equivalent positions. It is clear to the skilled person where the positions *ortho, meta* and/or *para* are located in a compound. Since the terms *ortho, meta* and *para* concern aromatic/aryl groups, the skilled person understands that non-aromatic cyclic groups can be substituted at positions that are equivalent to positions *ortho, meta* and *para* in aromatic/aryl groups. For example, a non-aromatic cyclic group as disclosed herein may be substituted at C₂, C₃ and/or C₄. Therefore, the substituted (C₆-C₁₀)-aryl may be substituted at positions *ortho, meta* and/or *para.* The substituted (C₅-C₈)-cycloalkenyl may be be substituted at C₂, C₃ and/or C₄.

It is further contemplated that the (C₆-C₁₀)-aryl or substituted (C₅-C₈)-cycloalkenyl is substituted with a compound selected from 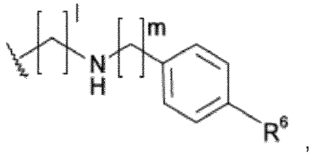 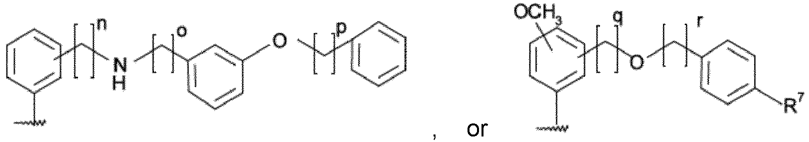
wherein "I", "m", "n", "o", "p", "q" and "r" are independently selected from 0 to 6;
R⁶ is selected from H, branched, cyclo or linear (C₁-C₆)-alkyl, or halogen;
R⁷is selected from halogen, NO₂, NH₂, CN, CF₃, branched, cyclo or linear (C₁-C₆)-alkyl.

It is also contemplated that R² is 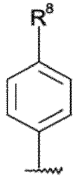 wherein R⁸ is selected from halogen, NO₂, NH₂, or CN.

The present invention also contemplates that R⁶ in formula (II) and/or R⁷ in formula (IV) can be located at position C2, C3 or C4 of the aryl.

The present invention also contemplates that the O in formula (III) is located at position C₂, C₃ or C₄ of the aryl.

The present invention also envisions that R² can be selected from

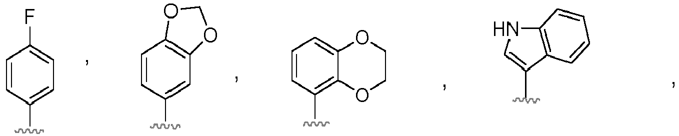

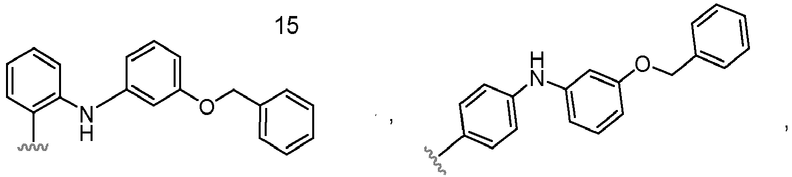

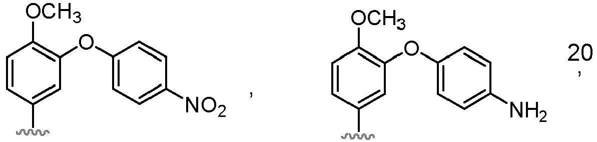

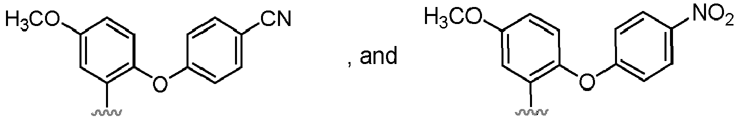

R² can also be selected from 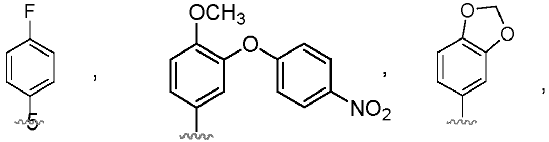 and

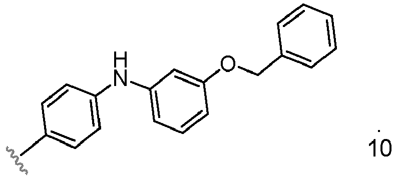

R² can also be selected from 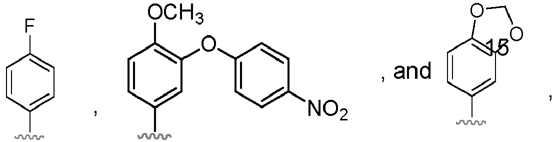 preferably

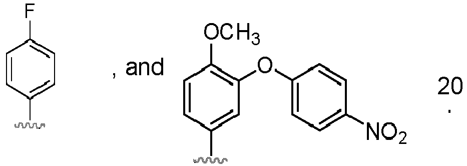

The halogen as disclosed herein can be any halogen. Exemplarily halogens areF, Cl, Br, and I. The halogen can also be F, Cl or I.

R₃ can independently be selected from H, branched, cyclo or linear (C₁-C₆)-alkyl, branched, cyclo or linear (C₁-C₆)-alkyl-OH, CF₃, halogen, NO₂, and CN. The present invention also contemplates that both R₃ substituents are the same or different.

R³ can thus, for example, be selected from H, methyl, ethyl, n-propyl, n-butyl, n-pentyl, n-hexyl, methyl-OH, ethyl-OH, propyl-OH, n-butyl-OH, n-pentyl-OH, n-hexyl-OH, methyl-NH₂, ethyl-NH₂, propyl-NH₂ *n*-butyl-NH₂, *n*-pentyl-NH₂, *n*-hexyl-NH₂, CF₃, halogen, NO₂, and CN. The halogen may be selected from F, Cl, Br, or from I, F, or Cl.

It is envisioned that R³ is selected from H branched, cyclo or linear (C₁-C₆)-alkyl, preferably methyl, and halogen, preferably F, Cl, and/or Br. It is also contemplated that R³ is selected from CH₃, OH, CF₃, Cl, Br, and/or F. The present invention also envisions that R³ can be selected from F, Cl, and/or Br. R³ can also be OH and/or CH₃. For example, one R³ substituent may be OH and the other one may be CH₃. It is also envisioned that both R³ substituents are either OH or CH₃.

R₄ can be independently selected from H, OH, O, NH₂, and NO₂. For example, R⁴ may be selected from O, OH and NH₂. It is envisioned that both R₄ substituents are the same or different.

The present invention also contemplates that R⁴ can be selected from O and/or OH. R⁴ can also be OH and/or CH₃. For example, one R⁴ substituent may be OH and the other one may be CH₃. It is also envisioned that both R⁴ substituents are either OH or CH₃. It is also envisioned that one R⁴ substituent may be OH and the other one may be O. It is also envisioned that both R⁴ substituents are either OH or O.

R⁵ can be independently selected from N, NH, O, S, branched, cyclo or linear (C₁-C₆)-alkyl, branched or linear (C₁-C₆)-alkyl comprising a secondary or tertiary amine, an ether group or a sulfide group, N=N, and CH=N. It is envisioned that both R₅ substituents are the same or different.

The skilled person understands that the branched or linear (C₁-C₆)-alkyl comprising a secondary amine in R⁵ has a formula of (C₁-C₅)-alkyl-NH-(C₁-C₅)-alkyl, or NH-(C₁-C₆)-alkyl. This means that the NH atom in R⁵ can have two substituents, namely two independently branched or linear (C₁-C₅)-alkyl groups as defined elsewhere herein. On the other hand, the NH atom in R⁵ may be part of the pyrrole of formula I so that the pyrrole becomes a pyrazole and may further comprise an alkyl group as substituent. The NH group of R⁵ can also be at the end of the alkyl chain. The NH group can therefore be positioned before C₁ (then the pyrrole of formula I becomes a pyrazole), between C₁ and C₂, C₂ and C₃, C₃ and C₄, C₄ and C₅ or C₅ and C₆ or on C₆ (or any other carbon atom which is the last one in the carbon chain). It is thus envisioned that the NH-group in R⁵ is located before C₁, between C₁ and C₂ and/or on C₆ (or any other carbon atom that is the last one on the alkyl chain).

The skilled person understands that the branched or linear (C₁-C₆)-alkyl comprising a tertiary amine in R⁵ has a formula of (C₁-C₄)-alkyl-N(C₁-C₄)-(C₁-C₄)-alkyl or N-(C₁-C₅)-alkyl-(C₁-C₅)-alkyl. This means that the N atom can have three substituents, namely three independently branched or linear (C₁-C₄)-alkyl groups as defined elsewhere herein. On the other hand, the N atom may be part of the pyrrole of formula I so that the pyrrole becomes a pyrazole and may further comprise two alkyl group as substituent. The N atom can also be at the end of the alkyl chain. The N atom can therefore be positioned before C₁ (then the pyrrole of formula I becomes a pyrazole), between C₁ and C₂, C₂ and C₃, C₃ and C₄, C₄ and C₅ or C₅ and C₆ or on C₆ (or any other carbon atom which is the last one in the carbon chain). It is thus envisioned that the N-group is located before C₁, between C₁ and C₂ and/or on C₆ (or any other carbon atom that is the last one on the alkyl chain).

The skilled person understands that the branched or linear (C₁-C₆)-alkyl comprising an ether group in R⁵ has a formula of (C₁-C₅)-alkyl-O-(C₁-C₅)-alkyl or O-(C₁-C₆)-alkyl. This means that the O atom can have two substituents, namely two independently branched or linear (C₁-C₅)-alkyl groups as defined elsewhere herein. On the other hand, the O atom in R⁵ may be part of the pyrrole of formula I so that the pyrrole becomes an isoxazole and may further comprise an alkyl group as substituent. The O atom can also be at the end of the alkyl chain. The O atom can therefore be positioned before C₁ (then the pyrrole of formula I becomes an isoxazole), between C₁ and C₂, C₂ and C₃, C₃ and C₄, C₄ and C₅ or C₅ and C₆ or on C₆ (or any other carbon atom which is the last one in the carbon chain). It is thus envisioned that the O atom is located before C₁, between C₁ and C₂ and/or on C₆ (or any other carbon atom that is the last one on the alkyl chain).

The branched or linear (C₁-C₆)-alkyl comprising a sulfide group as used herein refers to any branched or linear (C₁-C₆)-alkyl comprising a sulfide group.

The skilled person knows that the branched or linear (C₁-C₆)-alkyl comprising a sulfide has a formula of (C₁-C₅)-alkyl-S-(C₁-C₅)-alkyl, S-(C₀-C₆)-alkyl or (C₁-C₆)-alkyl-S. This means that the S atom can have two substituents, namely two independently branched or linear (C₁-C₅)-alkyl groups as defined elsewhere herein. On the other hand, the S atom may be part of the pyrrole of formula I so that the pyrrole becomes an isothiazole and may further comprise an alkyl group as substituent. The sulfide can also be at the end of the alkyl chain. The sulfide can therefore be positioned before C₁ (then the pyrrole of formula I becomes an isothiazole), between C₁ and C₂, C₂ and C₃, C₃ and C₄, C₄ and C₅ or C₅ and Ce or on C₆ (or any other carbon atom which is the last one in the carbon chain). It is thus envisioned that the S-atom is located before C₁, between C₁ and C₂ and/or on C₆ (or any other carbon atom that is the last one on the alkyl chain). (C₂)-alkyl comprising a sulfide can be methyl-S-methyl. (C₃)-alkyl comprising a sulfide can be selected from methyl-S-ethyl, ethyl-S-methyl. (C₄)-alkyl comprising a sulfide can be selected from methyl-S-propyl, ethyl-S-ethyl, propyl-S-methyl. (C₅)- alkyl comprising a sulfide can be selected from methyl-S-butyl, propyl-S-ethyl, ethyl-S-propyl, butyl-S-methyl. (C₆)- alkyl comprising a sulfide can be selected from methyl-S-pentyl, butyl-S-ethyl, propyl-S-propyl, ethyl-S-butyl, and pentyl-S-methyl. Preferably, the branched or linear (C₁-C₆)-alkyl comprising a sulfide is S-CH₃ or S-CH₂CH₃, wherein the S atom is positioned before C₁, so that the resulting ring is an isothiazole.

The present invention also envisions that R⁵ can be selected from N, O, NH, branched or linear (C₁-C₆)-alkyl comprising a secondary or tertiary amine, an ether group or a sulfide group, preferably the branched or linear (C₁-C₆)-alkyl comprising a secondary amine is N-CH₃.

It is also contemplated that R⁵ is N or NH. For example, one R⁵ substituent may be NH and the other one may be N. It is also envisioned that both R⁵ substituents are either NH or N.

The present invention also envisions that "I" can be selected from 0 to 3, preferably "I" is 0 ("I" is absent); "m" can be selected from 0 to 3, preferably "m" is 1, "n" can be selected from 0 to 3, preferably "n" is 0 ("n" is absent); "o" can be selected from 0 to 3, preferably "o" is 0 ("o" is absent); "p" can be selected from 0 to 3, preferably "p" is 1; "q" can be selected from 0 to 3, preferably "q" is 0 ("q" is absent); "r" can be selected from 0 to 3, preferably "r" is 0 ("r" is absent).

The present invention also relates to the following compounds

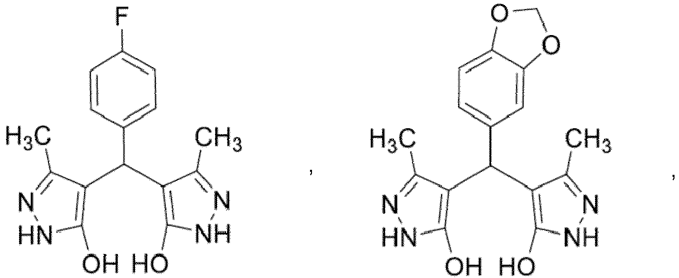

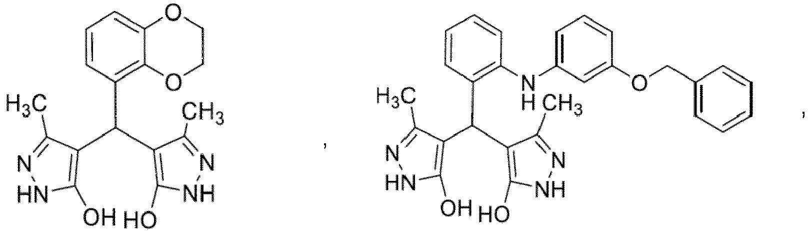

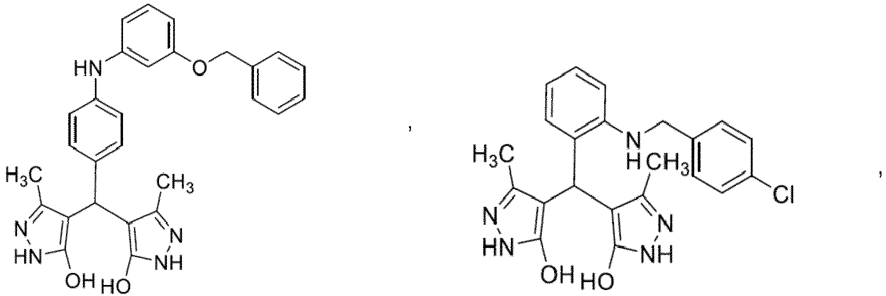

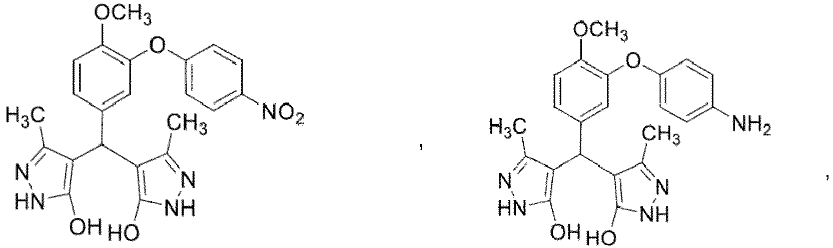

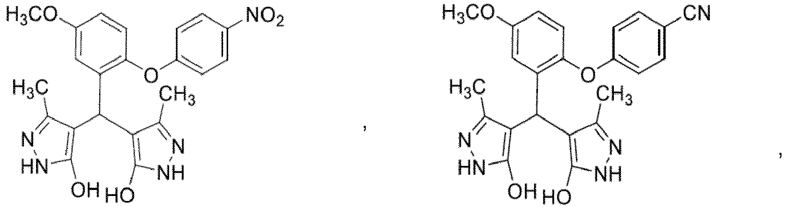

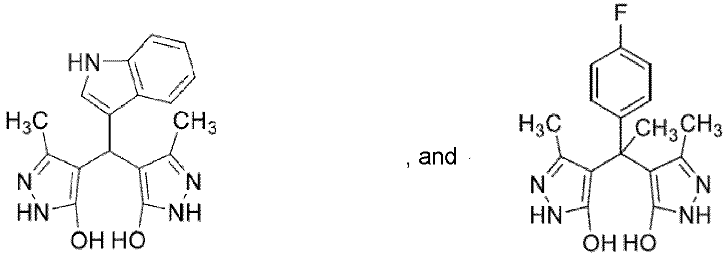

The present invention also envisions the following compounds 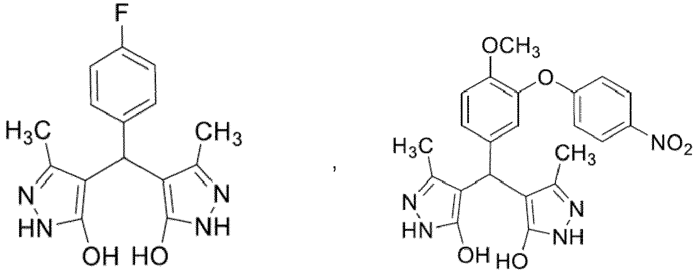 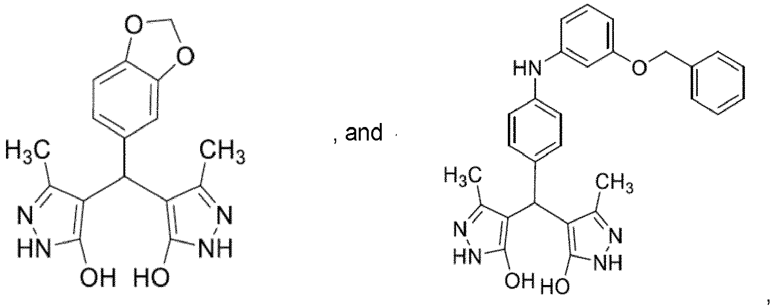 preferably compounds 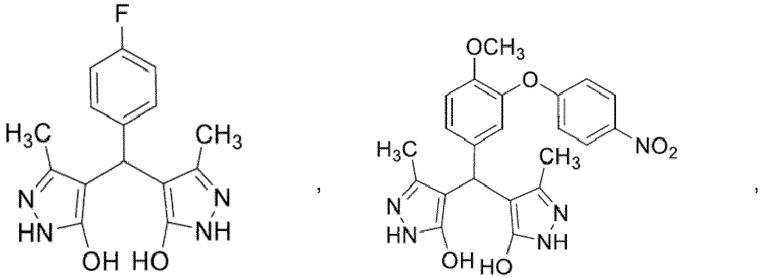 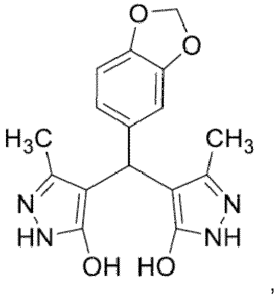 more preferably compounds

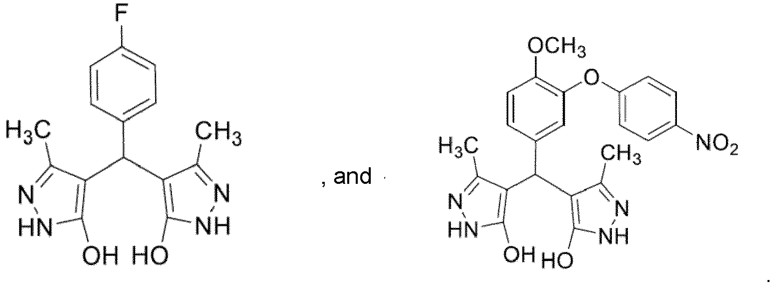

The compound can also be any one of P14A to P14D as shown in Fig. 2, preferably, the compound is P14A, P14B or P14C as shown in Fig. 2, most preferably, the compound is P14A or P14B as shown in Fig. 2

The present invention also relates to intermediates compounds that can be used in the synthesis of the compounds of the present invention. Specifically, the intermediate compound is selected from
a) branched, cyclo or linear (C₁-C₆)-alkyl substituted with CHO;
b) heterocycle substituted with CHO;
c) (C₅-C₈)-cycloalkenyl substituted with CHO;
d) (C₆-C₁₀)-aryl substituted with CHO;
e) (C₅-C₈)-cycloalkenyl, or the (C₆-C₁₀)-aryl substituted with CHO and with 1 or 2 groups selected from
   i) OCH₃;
   ii) halogen;
   iii) a substituent having formula II 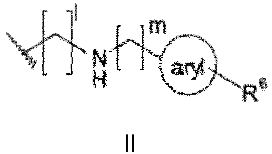 wherein "I" and "m" are independently selected from 0 to 6, the aryl group has 5 to 10 atoms, and wherein R⁶ is H, branched, cyclo or linear (C₁-C₆)-alkyl, or halogen;
   iv) a substituent having formula III 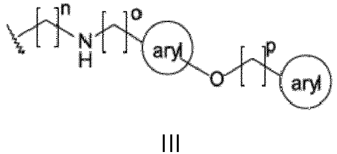 wherein the aryl groups independently have 5 to 10 atoms, and "n", "o" and "p" are independently selected from 0 to 6; and/or
   v) a substituent having formula IV 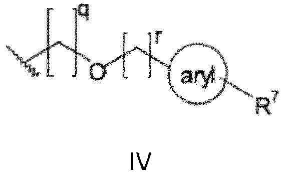 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.

It is further contemplated that the intermediate compound can be selected from a heterocycle substituted with CHO, 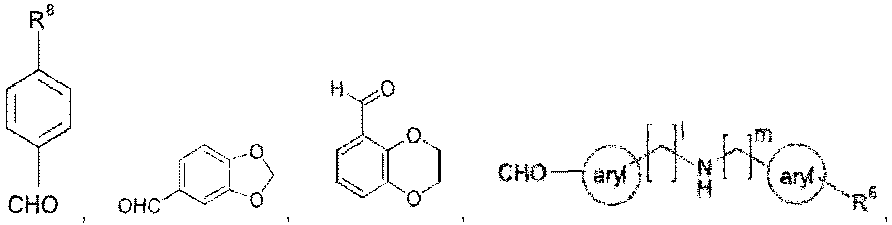 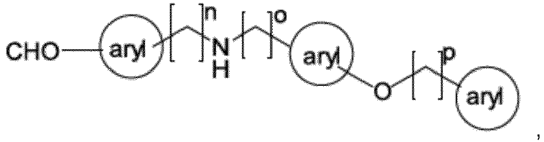 and 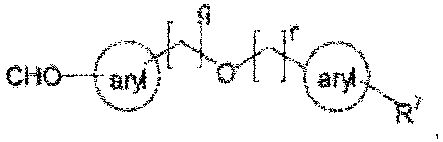
wherein "I", "m", "n", "o", "q" and "r" are independently selected from 0 to 6, the aryl group has 5 to 10 atoms,
R⁶ is H, branched, cyclo or linear (C₁-C₆)-alkyl, or halogen; and
R⁷ is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.

The intermediate compound may thus be selected from

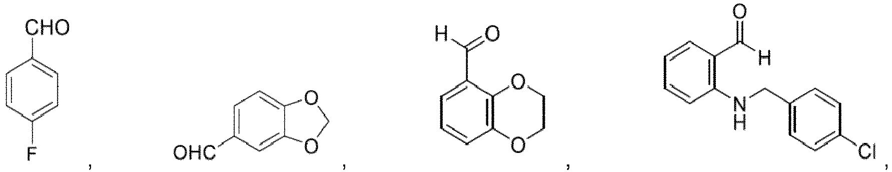

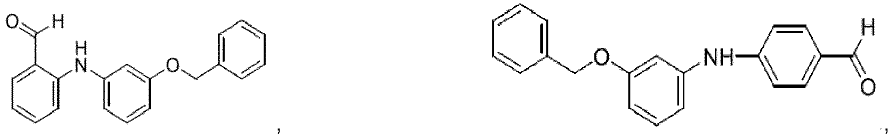

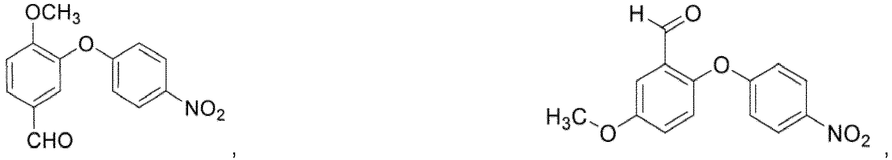

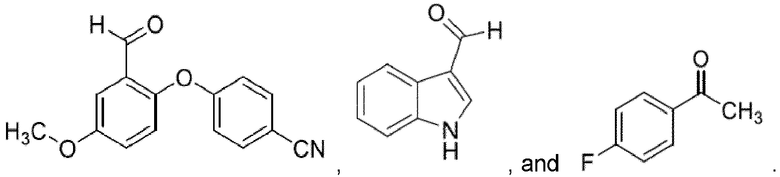

The present invention also relates to the compound of the present invention or a salt or solvate thereof or a compound of formula 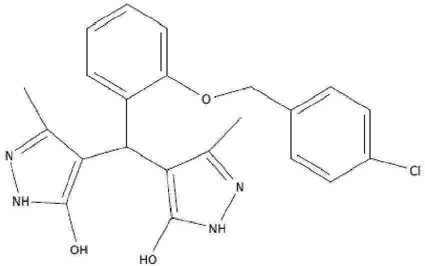 or a salt or solvate thereof
for use as a medicament. For example, these compounds may be used in treating or preventing pancreatic cancer, lung cancer, colorectal cancer, thyroid cancer, testicle cancer, melanoma, bladder cancer, liver cancer, kidney cancer, myelodysplastic syndrome, or leukemia.

The present invention also concerns a pharmaceutical composition comprising the compound of the present invention or a pharmaceutically acceptable salt or solvate thereof or a compound of formula 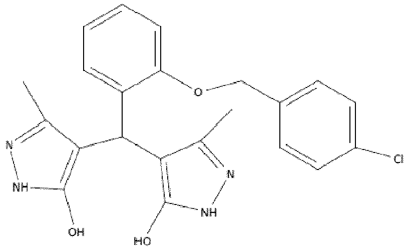 or salt or solvate thereof.

The salt or solvate may be any suitable salt or solvate. For example, the salt may be a pharmaceutically acceptable salt. The term "pharmaceutically acceptable salt" refers to any pharmaceutically acceptable salt, which upon administration to the patient is capable of providing (directly or indirectly) a compound as described herein. Such salts preferably are acid addition salts with physiologically acceptable organic or inorganic acids, or alkali addition salts with acceptable organic or inorganic bases. Examples of the acid addition salts include mineral acid addition salts such as, for example, hydrochloride, hydrobromide, hydroiodide, sulphate, nitrate, phosphate, and organic acid addition salts such as, for example, lactate, acetate, trifluoroacetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium and ammonium salts, and organic alkali salts such as, for example, ethylenediamine, ethanolamine, *N,N*-dialkylenethanolamine, triethanolamine and basic aminoacids salts. However, it will be appreciated that non-pharmaceutically acceptable salts also fall within the scope of the invention since those may be useful in the preparation of pharmaceutically acceptable salts. Procedures for salt formation are conventional in the art.

The term "pharmaceutically acceptable solvate" in accordance with this invention should be understood as meaning any form of the active compound in accordance with the invention in which said compound is bonded by a non-covalent bond to another molecule (normally a polar solvent), including especially hydrates and alcoholates.

The pharmaceutical composition of the present invention may be used as a medicament. For example, the pharmaceutical composition of the present invention may be used in treating pancreatic cancer, lung cancer, colorectal cancer, thyroid cancer, testicle cancer, melanoma, bladder cancer, liver cancer, kidney cancer, myelodysplastic syndrome, or leukemia.

It is further contemplated that the pharmaceutical composition further comprises an excipient, carrier or solvent. Such compositions can be included in a capsule, sachet, paper or other container. In making the compositions, conventional techniques for the preparation of pharmaceutical compositions may be used. For example, the compounds for use according to present invention may be mixed with a carrier, or diluted by a carrier, or enclosed within a carrier that may be in the form of an ampoule, capsule, sachet, paper, or other container. When the carrier serves as a diluent, it may be solid, semisolid, or liquid material that acts as a vehicle, excipient, or medium for the active compound. The compounds for use according to present invention can be adsorbed on a granular solid container for example in a sachet. Some examples of suitable carriers are water, salt solutions, alcohols, polyethylene glycols, polyhydroxyethoxylated castor oil, peanut oil, olive oil, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose, and polyvinylpyrrolidone. Similarly, the carrier or diluent may include any sustained release material known in the art, such as glyceryl monostearate or glyceryl distearate, alone or mixed with a wax. Said compositions may also include wetting agents, emulsifying and suspending agents, preserving agents, sweetening agents or flavoring agents. The compositions for use according to the invention may be formulated so as to provide quick, sustained, or delayed release of the compounds for use according to present invention after administration to the patient by employing procedures well known in the art.

The pharmaceutical compositions can be sterilized and mixed, if desired, with auxiliary agents, emulsifiers, salt for influencing osmotic pressure, buffers and/or coloring substances and the like, which do not deleteriously react with the compounds for use according to present invention.

In addition to the compounds for use according to present invention, the compositions for use according to the invention may include, depending on the composition and mode of delivery desired, pharmaceutically acceptable, non-toxic carriers or diluents, which include vehicles commonly used to form pharmaceutical compositions for animal or human administration. The diluents are selected so as not to unduly affect the biological activity of the combination.

The compound of the present invention and/or the pharmaceutical composition of the present invention may be administered to a subject. Preferably, the subject is a subject in need of such administration. The subject may be any suitable subject. The subject may be a human or an animal. Preferably the subject is a human.

One preferred embodiment disclosed herein refers to the route of administration, that may be any route which effectively transports the compounds and compositions disclosed in present disclosure, to the appropriate or desired site of action, such as oral, nasal, topical, pulmonary, transdermal, intrathecal or parenteral, e. g., rectal, subcutaneous, intravenous, intraurethral, intramuscular, intranasal, ophthalmic solution or an ointment.

For oral administration, either solid or fluid unit dosage forms can be prepared. For preparing solid compositions such as tablets, the compounds for use according to present invention, are mixed into formulations with conventional ingredients such as talc, magnesium stearate, di-calcium phosphate, magnesium aluminum silicate, calcium sulphate, starch, lactose, acacia, methylcellulose, and functionally similar materials as pharmaceutical diluents or carriers.

Capsules are prepared by mixing the compounds for use according to present invention with an inert pharmaceutical diluent and filling the mixture into a hard gelatin capsule of appropriate size. Soft gelatin capsules are prepared by machine encapsulation of slurry of the compound with an acceptable vegetable oil, light liquid petrolatum or other inert oil. Fluid unit dosage forms for oral administration such as syrups, elixirs and suspensions can be prepared. The water-soluble forms can be dissolved in an aqueous vehicle together with sugar, aromatic flavoring agents and preservatives to form syrup. An elixir is prepared by using a hydroalcoholic (e. g., ethanol) vehicle with suitable sweeteners such as sugar and saccharin, together with an aromatic flavoring agent.

Suspensions can be prepared with an aqueous vehicle with the aid of a suspending agent such as acacia, tragacanthin, methylcellulose and the like.

Examples of such diluents that are especially useful for injectable formulations are water, the various saline, organic or inorganic salt solutions, Ringer's solution, dextrose solution, and Hank's solution. In addition, the pharmaceutical composition or formulation may include additives such as other carriers; adjuvants; or non-toxic, non-therapeutic, non-immunogenic stabilizers and the like.

Furthermore, excipients can be included in the compositions disclosed. Examples include, but are not limited to, cosolvents, surfactants, oils, humectants, emollients, preservatives, stabilizers and antioxidants. Any pharmacologically acceptable buffer may be used, such as, tris or phosphate buffers. Effective amounts of diluents, additives, and excipients are those amounts that are effective to obtain a pharmaceutically acceptable formulation in terms of solubility, biological activity, etc.

The pharmaceutical compositions comprising the compounds for use according to present invention may be incorporated into a microsphere. The compounds for use according to present invention can be loaded into albumin microspheres, from which it is possible to recover such microspheres in a dry powder for nasal administration. Other materials suitable for the preparation of microspheres include agar, alginate, chitosan, starch, hydroxyethyl starch, albumin, agarose, dextran, hyaluronic acid, gelatin, collagen, and casein. The microspheres can be produced by various processes known to the person skilled in the art such as a spray drying process or an emulsification process.

Another preferred embodiment of the invention is the dosage scheme of the compounds for use according to present invention. The term "unit dosage form" refers to physically discrete units suitable as unitary dosages for subjects, e. g., mammalian subjects, e. g. humans, dogs, cats, and rodents, each unit containing a predetermined quantity of active material calculated to produce the desired pharmaceutical effect in association with the required pharmaceutical diluent, carrier or vehicle. The specifications for the unit dosage forms of this invention are dictated by and dependent on (a) the unique characteristics of the compounds and extracts disclosed above herein and the particular effect to be achieved and (b) the limitations inherent in the art wherein said compounds or extracts are used in humans and animals. Examples of unit dosage forms are tablets, capsules, pills, powder packets, wafers, suppositories, granules, cachets, teaspoonfuls, tablespoonfuls, dropperfuls, ampoules, vials, aerosols with metered discharges, segregated multiples of any of the foregoing, and other forms as herein described. The compositions disclosed herein can be included in kits, which can contain one or more-unit dosage forms of the composition and instructions for use.

Slow or extended-release delivery systems, including any of a number of biopolymers (biological-based systems), systems employing liposomes, colloids, resins, and other polymeric delivery systems or compartmentalized reservoirs, can be utilized with the compositions described herein to provide a continuous or long-term source of the therapeutic compound.

The compound of the present invention, the compound for use of the present invention as well as the pharmaceutical composition of the present invention and the use of the pharmaceutical composition of the present invention can have different effects. For example, the compound or the pharmaceutical composition of the present invention may increase RAS signaling. For example, the compound/pharmaceutical composition increases RAS signaling compared to a control. The increase in RAS signaling may be detected by
a) seeding DLD-1 KRAS ^{WT/G13D} clone V15 endogenously expressing oncogenic KRAS or DLD-1 KO KRAS ^{WT/+} clone DWT7 cells stably expressing HA-KRAS G12V in media comprising 10 % FBS for 24 hours;
b) serum starving the cells for 24 hours;
c) incubating the cells for 3 hours with 100 µM of the compound, for 30 minutes with 10 % FBS as positive control and 0 % FBS as negative control;
d) lysing the cells in a buffer containing 67 mM Tris-HCl and 2 % SDS having a pH of 6.8 and then incubating at 97°C for 15 minutes;
e) determining the protein concentration of the lysates using the Lowry method;
f) resolving 15 µg of protein on SDS-PAGE
g) transferring the separated proteins onto a PVDF membrane;
h) detecting activation of Ras effector proteins ERK and AKT using anti-pAKT or anti-pERK antibodies;
i) comparing the signal obtained for the compound to the signal obtained for the 0 % FBS negative control and 10% FBS as positive control;
j) detecting an increased signal for the compound of p-AKT and p-ERK compared to the negative control..

Additionally, or alternatively, the compound or the pharmaceutical composition of the present invention may decrease tumor cell viability. Tumor cell viability may be decreased compared to a control. The decrease in tumor cell viability may be detected by
a) culturing 10000 DLD-1 KRAS ^{WT/G13D} clone V15 cells expressing endogenous oncogenic KRAS in 50 µl of a medium comprising 10 % FBS in a 96-well plate for 24 hours;
b) adding 50 µl of the compound in media or 50 µl of medium as negative control to the culturing of step a);
c) performing an MTS viability assay;
d) calculating the percentage of viability by dividing the absorbance of each well by the average absorbance of the negative control wells;
e) detecting a decreased viability of the cells compared to control cells not treated with the compound.

It is further contemplated that the compound of the present invention interacts with Glu168 and Lys169 of KRAS of SEQ ID No. 1 or SEQ ID NO. 2. For example, the compound may interact with GST-KRAS-G12V (oncogenic KRAS). The interaction of the compound with GST-KRAS-G12V can for example be detected via plasmon resonance analysis. The GST-KRAS-G12V construct is described in Lopez-Alcalá C, et al. J Biol Chem. 2008 Apr 18;283(16):10621-31. doi: 10.1074/jbc.M706238200.

It is further envisioned that the compound of the present invention competes with Calmodulin (CaM) of SEQ ID No. 3 for binding to amino acids 1 to 160 of oncogenic GST-KRAS-G12V of SEQ ID No. 2. The competing may be detected by CaM-sepharose pulldown assay using GST-KRAS-G12V in the presence of the compound of the present invention.

It is also contemplated that the compound of the present invention increases HA-KRAS G12V interaction with BRAF and P-C-RAF S338 compared to a control. For example, the compound may increase RAS signaling of cells expressing KRAS G12V of SEQ ID No. 2 compared to control.

It is further envisioned that the compound of the present invention reduces the cell viability of DLD-1 cells to a larger extend than erlotinib, wherein the compound and erlotinib are used in the same concentration.

The present invention also relates to a method for the preparation of a of the present invention or the pharmaceutical composition of the present invention, the process comprising contacting 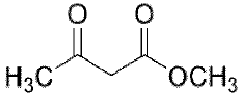 NH₂-NH₂·H₂O with and with one intermediate compound of the present invention as defined herein.

The present invention also relates to a kit or package comprising the compound of the present invention or the pharmaceutical composition of the present invention. The kit or package may further comprise instructions for the medical use of the compound of the present invention or of the pharmaceutical composition of the present invention.

Alternatively, or additionally, the kit or package may comprise a unit dosage form of the compound of the present invention, or of the pharmaceutical composition of the present invention.

The present invention also relates to a use of a compound of the present invention or a salt or solvate thereof, or the pharmaceutical composition of the present invention, for the preparation of a medicament.

The present invention also concerns a use of a compound of the present invention or a salt or solvate thereof or the pharmaceutical composition of the present invention for the preparation of a medicament for treating pancreatic cancer, lung cancer, colorectal cancer, thyroid cancer, testicle cancer, melanoma, bladder cancer, liver cancer, kidney cancer, myelodysplastic syndrome, or leukemia.

The present invention also relates to a method of treating a disease, the method comprising administering to a subject in need thereof a therapeutically effective amount of a compound of the present invention or a salt or solvate thereof, or a pharmaceutical composition of the present invention.

The present invention further concerns a method of treating pancreatic cancer, lung cancer, colorectal cancer, thyroid cancer, testicle cancer, melanoma, bladder cancer, liver cancer, kidney cancer, myelodysplastic syndrome, or leukemia, the method comprising administering to a subject in need thereof a therapeutically effective amount of a compound of the present invention or a salt or solvate thereof, or a pharmaceutical composition of the present invention.

The present invention is further characterized by the following items.
1. A compound having the general formula I 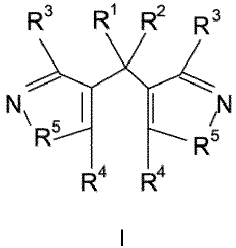 wherein
   R¹ is selected from H, branched, cyclo or linear (C₁-C₆)-alkyl, branched, cyclo or linear (C₁-C₆)-alkyl-OH, branched, cyclo or linear (C₁-C₆)-alkyl-NH₂, optionally substituted with one or more halogens, branched or linear (C₁-C₆)-alkyl comprising a secondary or tertiary amine or an ether group;
   R² is selected from branched, cyclo or linear (C₁-C₆)-alkyl, heterocyclyl, heteroaryl, substituted or unsubstituted (C₆-C₁₀)-aryl, or substituted or unsubstituted (C₅-C₈)-cycloalkenyl,
   wherein the substituted (C₆-C₁₀)-aryl or substituted (C₅-C₈)-cycloalkenyl is substituted with 1 or 2 groups selected from the groups consisting of
      a) OCH₃,
      b) halogen,
      c) a substituent having formula II 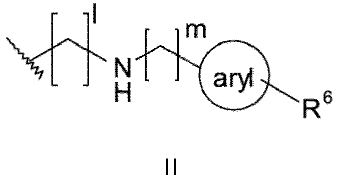 wherein "I" and "m" are independently selected from 0 to 6, the aryl group has 5 to 10 atoms, and wherein R⁶ is H, branched, cyclo or linear (C₁-C₆)-alkyl, or halogen;
      d) a substituent having formula III 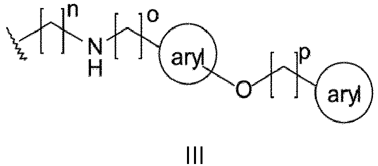 wherein the aryl groups independently have 5 to 10 atoms, and "n", "o" and "p" are independently selected from 0 to 6; and/or
      e) a substituent having formula IV 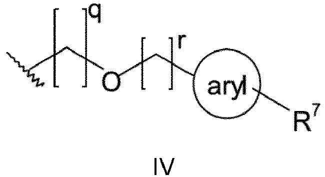 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1;
   R₃ is independently selected from H, branched, cyclo or linear (C₁-C₆)-alkyl, branched, cyclo or linear (C₁-C₆)-alkyl-OH, CF₃, halogen, NO₂, and CN;
   R₄ is independently selected from H, OH, O, NH₂, and NO₂; and
   R⁵ is independently selected from N, NH, O, S, branched, cyclo or linear (C₁-C₆)-alkyl, branched or linear (C₁-C₆)-alkyl comprising a secondary or tertiary amine, an ether group or a sulfide group, N=N, and CH=N.
2. The compound of item 1, wherein the branched, cyclo or linear (C₁-C₆)-alkyl is selected from
   (C₁)-alkyl is methyl;
   (C₂-alkyl is ethyl;
   (C₃)-alkyl is selected from the group consisting of propyl and cyclopropyl;
   (C₄)-alkyl is selected from the group consisting of n-butyl, isopropyl, butan-2-yl, 2-methylpropyl, tert-butyl, cyclobutyl and methylcyclopropyl;
   (C₅)-alkyl is selected from the group consisting of n-pentyl, 2-methylbutyl, 2,2-dimethylpropyl, 3-methylbutyl, pentan-2-yl, pentan-3-yl, 3-methylbutan-2-yl, 2-methylbutyl, cyclopentyl, methylcyclobutyl, 1,1-dimethylcyclopropyl and 1,2-dimethylcyclopropyl; and
   (C₆)-alkyl is selected from the group consisting of n-hexyl, 2-methylpentyl, 3-methylpentyl, 2,2-dimethylbutyl, 2,3-dimethylbutyl, cyclohexyl, methylcyclopentyl, 1,2-dimethylcyclobutyl, 1,3-dimethylcyclobutyl and 1,2,3-trimethylcyclopropyl.
3. The compound of item 1 or 2, wherein the branched, cyclo or linear (C₁-C₆)-alkyl is selected from
   (C₁)-alkyl is methyl;
   (C₂-alkyl is ethyl;
   (C₃)-alkyl is propyl;
   (C₄)-alkyl is selected from the group consisting of n-butyl, isopropyl, butan-2-yl, 2-methylpropyl, tert-butyl, preferably n-butyl;
   (C₅)-alkyl is selected from the group consisting of n-pentyl, 2-methylbutyl, 2,2-dimethylpropyl, 3-methylbutyl, pentan-2-yl, pentan-3-yl, 3-methylbutan-2-yl, 2-methylbutyl, preferably n-pentyl; and
   (C₆)-alkyl is selected from the group consisting of n-hexyl, 2-methylpentyl, 3-methylpentyl, 2,2-dimethylbutyl, 2,3-dimethylbutyl, preferably n-hexyl.
4. The compound of any one of items 1 to 3, wherein the branched, cyclo or linear
   (C₁-C₆)-alkyl-OH is selected from
   (C₁)-alkyl-OH is methyl-OH;
   (C₂)-alkyl-OH is ethyl-OH;
   (C₃)-alkyl-OH is selected from the group consisting of propyl-OH and cyclopropyl-OH;
   (C₄)-alkyl-OH is selected from the group consisting of n-butyl-OH, isopropyl-OH, butan-2-yl-OH, 2-methylpropyl-OH, tert-butyl-OH, cyclobutyl-OH and methylcyclopropyl-OH; (C₅)-alkyl-OH is selected from the group consisting of n-pentyl-OH, 2-methylbutyl-OH, 2,2-dimethylpropyl-OH, 3-methylbutyl-OH, pentan-2-yl-OH, pentan-3-yl-OH, 3-methylbutan-2-yl-OH, 2-methylbutyl-OH, cyclopentyl-OH, methylcyclobutyl-OH, 1,1-dimethylcyclopropyl-OH and 1,2-dimethylcyclopropyl-OH; and
   (C₆)-alkyl-OH is selected from the group consisting of n-hexyl-OH, 2-methylpentyl-OH, 3-methylpentyl-OH, 2,2-dimethylbutyl-OH, 2,3-dimethylbutyl-OH, cyclohexyl-OH, methylcyclopentyl-OH, 1,2-dimethylcyclobutyl-OH, 1,3-dimethylcyclobutyl-OH and 1,2,3-trimethylcyclopropyl-OH.
5. The compound of any one of items 1 to 4, wherein the branched, cyclo or linear
   (C₁-C₆)-alkyl-OH is selected from
   (C₁)-alkyl-OH is methyl-OH;
   (C₂)-alkyl-OH is ethyl-OH;
   (C₃)-alkyl-OH is propyl-OH;
   (C₄)-alkyl-OH is selected from the group consisting of n-butyl-OH, isopropyl-OH, butan-2-yl-OH, 2-methylpropyl-OH, tert-butyl-OH, preferably n-butyl-OH;
   (C₅)-alkyl-OH is selected from the group consisting of n-pentyl-OH, 2-methylbutyl-OH, 2,2-dimethylpropyl-OH, 3-methylbutyl-OH, pentan-2-yl-OH, pentan-3-yl-OH, 3-methylbutan-2-yl-OH, 2-methylbutyl-OH, preferably n-pentyl-OH; and
   (C₆)-alkyl-OH is selected from the group consisting of n-hexyl-OH, 2-methylpentyl-OH, 3-methylpentyl-OH, 2,2-dimethylbutyl-OH, 2,3-dimethylbutyl-OH, preferably n-hexyl-OH.
6. The compound of any one of items 1 to 5, wherein the branched, cyclo or linear
   (C₁-C₆)-alkyl-OH is selected from
   (C₁)-alkyl-NH₂ is methyl-NH₂;
   (C₂)-alkyl-OH is ethyl-NH₂;
   (C₃)-alkyl-OH is selected from the group consisting of propyl-NH₂ and cyclopropyl-NH₂;
   (C₄)-alkyl-OH is selected from the group consisting of *n*-butyl-NH₂, isopropyl-NH₂, butan-2-yl-NH₂, 2-methylpropyl-NH₂, tert-butyl- NH₂, cyclobutyl-NH₂ and methylcyclopropyl-NH₂;
   (C₅)-alkyl-NH₂ is selected from the group consisting of *n*-pentyl-NH₂, 2-methylbutyl-NH₂, 2,2-dimethylpropyl-NH₂, 3-methylbutyl-NH₂, pentan-2-yl-NH₂, pentan-3-yl-NH₂, 3-methylbutan-2-yl-NH₂, 2-methylbutyl-NH₂, cyclopentyl-NH₂, methylcyclobutyl-NH₂, 1,1-dimethylcyclopropyl- NH₂ and 1,2-dimethylcyclopropyl-NH₂; and
   (C₆)-alkyl-NH₂ is selected from the group consisting of *n*-hexyl-NH₂, 2-methylpentyl-NH₂, 3-methylpentyl-NH₂, 2,2-dimethylbutyl-NH₂, 2,3-dimethylbutyl-NH₂, cyclohexyl-NH₂, methylcyclopentyl-NH₂, 1,2-dimethylcyclobutyl-NH₂, 1,3-dimethylcyclobutyl-NH₂ and 1,2,3-trimethylcyclopropyl-NH₂.
7. The compound of any one of items 1 to 6, wherein the branched, cyclo or linear
   (C₁-C₆)-alkyl-OH is selected from
   (C₁)-alkyl-NH₂ is methyl-NH₂;
   (C₂)-alkyl- NH₂ is ethyl-NH₂;
   (C₃)-alkyl- NH₂ is propyl-NH₂;
   (C₄)-alkyl- NH₂ is selected from the group consisting of *n*-butyl-NH₂, isopropyl-NH₂, butan-2-yl-NH₂, 2-methylpropyl-NH₂, tert-butyl- NH₂, preferably *n*-butyl-NH₂;
   (C₅)-alkyl-NH₂ is selected from the group consisting of *n*-pentyl-NH₂, 2-methylbutyl-NH₂, 2,2-dimethylpropyl-NH₂, 3-methylbutyl-NH₂, pentan-2-yl-NH₂, pentan-3-yl-NH₂, 3-methylbutan-2-yl-NH₂, 2-methylbutyl-NH₂, preferably *n*-pentyl-NH₂; and
   (C₆)-alkyl-NH₂ is selected from the group consisting of *n*-hexyl-NH₂, 2-methylpentyl-NH₂, 3-methylpentyl-NH₂, 2,2-dimethylbutyl-NH₂, 2,3-dimethylbutyl-NH₂, preferably n-hexyl-NH₂.
8. The compound of any one of items 1 to 7, wherein the branched, cyclo or linear
   (C₁-C₆)-alkyl comprising a secondary or tertiary amine or an ether group is selected from (C₂)-alkyl comprising a secondary amine or an ether group is methyl-O-methyl or methyl-NH-methyl;
   (C₃)-alkyl comprising a secondary amine or an ether group is selected from methyl-O-ethyl, ethyl-O-methyl, methyl-NH-ethyl or ethyl-NH-methyl;
   (C₄)-alkyl comprising a secondary amine or an ether group is selected from methyl-O-propyl, ethyl-O-ethyl, propyl-O-methyl, methyl-NH-propyl, ethyl-NH-ethyl, propyl-NH-methyl;
   (C₅)-alkyl comprising a secondary amine or an ether group is selected from methyl-O-butyl, propyl-O-ethyl, ethyl-O-propyl, butyl-O-methyl, methyl-NH-butyl, propyl-NH-ethyl, ethyl-NH-propyl, butyl-NH-methyl;
   (C₆)-alkyl comprising a secondary amine or an ether group is selected from methyl-O-pentyl, butyl-O-ethyl, propyl-O-propyl, ethyl-O-butyl, pentyl-O-methyl, methyl-NH-pentyl, butyl-NH-ethyl, propyl-NH-propyl, ethyl-NH-butyl, pentyl-NH-methyl.
9. The compound of any one of items 1 to 8, wherein R¹ is selected from H and CH₃.
10. The compound of any one of items 1 to 9, wherein the heteroaryl is selected from indolyl, pyrrolyl, furanyl, thiophenyl, pyridyl, pyrimidyl, pyridazyl, pyrazinyl, quinolinyl, isoquinolinyl, acridinyl, 1,2-methylenedioxyphenyl or 1,2-ethylenedioxyphenyl, preferably 1-*H*-indolyl, 1,2-methylenedioxyphenyl and 1,2-ethylenedioxyphenyl.
11. The compound of any one of items 1 to 10, wherein the substituted or unsubstituted (C₅-C₈)-cycloalkenyl is selected from cyclopentenyl, cyclopentadienyl, cyclohexenyl, 1,3-cyclohexadiene, cycloheptenyl, 1,3-cycloheptadienyl, cycloheptatrienyl, cyclooctenyl, 1,5-cyclooctadienyl, or cyclooctatetraenyl.
12. The compound of any one of items 1 to 11, wherein the substituted or unsubstituted (C₆-C₁₀)-aryl is selected from substituted or unsubstituted phenyl, substituted or unsubstituted tolyl, substituted or unsubstituted xylyl, and substituted or unsubstituted naphthyl, preferably substituted phenyl.
13. The compound of any one of items 1 to 12, wherein R² is indol-2- or 3-yl, pyrrol-3-yl, substituted phenyl, 1,2-methylenedioxyphenyl, 1,2-ethylenedioxyphenyl, 2- or 3-pyrrolyl, 2- or 3-furanyl, 2- or-thiophenyl, 2-, 3- or 4-pyridyl, 2- 4 or 6-pyrimidyl, quinolinyl, isoquinolinyl.
14. The compound of any one of items 1 to 13, wherein the aryl group has 5 to 10 atoms in formulae (II), (III) and/or (IV) is selected from phenyl, pyrrolyl, furanyl, thiophenyl, pyridyl, pyrimidyl, quinolinyl and/or isoquinolinyl, preferably phenyl.
15. The compound of any one of items 1 to 14, wherein the (C₆-C₁₀)-aryl or substituted (C₅-C₈)-cycloalkenyl is substituted with OCH₃ and a substituent having formula IV 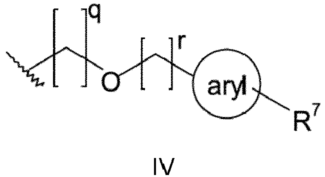 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.
16. The compound of any one of items 1 to 15, wherein R⁶ in formula (II) and/or R⁷ in formula (IV) are located at position C2, C3 or C4 of the aryl.
17. The compound of any one of items 1 to 16, wherein the O in formula (III) is located at position C2, C3 or C4 of the aryl.
18. The compound of any one of items 1 to 17 wherein the substituted (C₆-C₁₀)-aryl or substituted (C₅-C₈)-cycloalkenyl is substituted at positions *ortho, meta* and/or *para* or equivalent position.
19. The compound of any one of items 1 to 18 wherein the substituted (C₆-C₁₀)-aryl or substituted (C₅-C₈)-cycloalkenyl is substituted at positions *ortho, meta* and/or *para* or equivalent position.
20. The compound of any one of items 1 to 19, wherein the (C₆-C₁₀)-aryl or substituted (C₅-C₈)-cycloalkenyl is substituted with a compound selected from 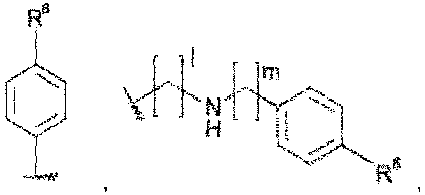 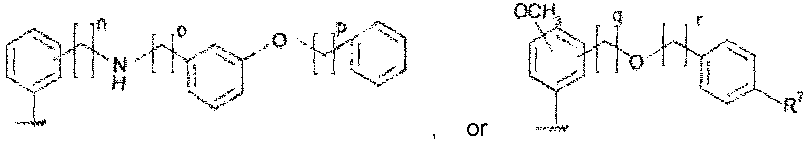
   wherein "I", "m", "n", "o", "p", "q" and "r" are independently selected from 0 to 6;
   R⁶ is selected from H, branched or linear (C₁-C₆)-alkyl, or halogen;
   R⁷is selected from halogen, NO₂, NH₂, CN, CF₃, branched, cyclo or linear (C₁-C₆)-alkyl;
   R⁸ is selected from halogen, NO₂, NH₂, or CN.
21. The compound of any one of items 1 to 20, wherein R² is selected from 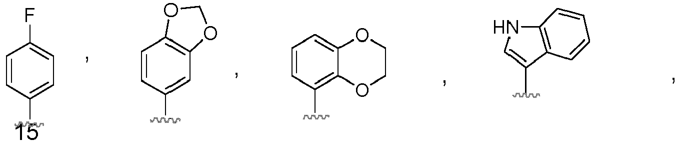 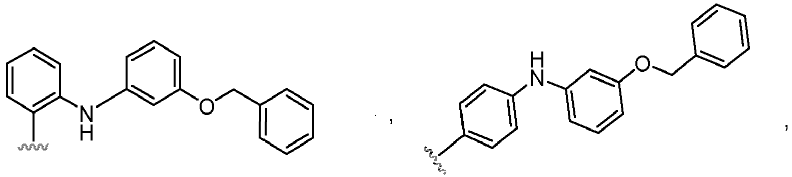 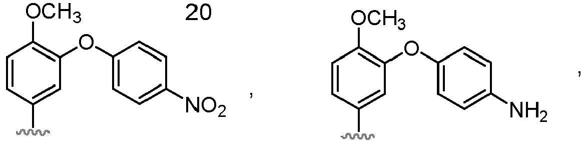 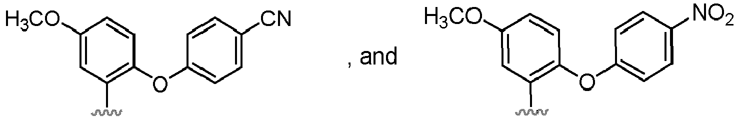
22. The compound of any one of items 1 to 21, wherein the halogen is selected from F, Cl, Br, and I, preferably F, Cl or I.
23. The compound of any one of items 1 to 22, wherein R³ is selected from CH₃, OH, CF₃, Cl, Br, or F.
24. The compound of any one of items 1 to 23, wherein R⁴ is selected from O, OH and NH₂.
25. The compound of any one of items 1 to 24, wherein R⁵ is selected from O, NH, branched or linear (C₁-C₆)-alkyl comprising a secondary or tertiary amine, an ether group or a sulfide group , preferably branched or linear (C₁-C₆)-alkyl comprising a secondary amine is N-CH₃.
26. The compound of any one of items 1 to 25, wherein "I" is selected from 0 to 3, preferably "I" is 0 ("I" is absent).
27. The compound of any one of items 1 to 16, wherein "m" is selected from 0 to 3, preferably "m" is 1.
28. The compound of any one of items 1 to 27, wherein "n" is selected from 0 to 3, preferably "n" is 0 ("n" is absent).
29. The compound of any one of items 1 to 28, wherein "o" is selected from 0 to 3, preferably "o" is 0 ("o" is absent).
30. The compound of any one of items 1 to 29, wherein "p" is selected from 0 to 3, preferably "p" is 1.
31. The compound of any one of items 1 to 30, wherein "q" is selected from 0 to 3, preferably "q" is 0 ("q" is absent).
32. The compound of any one of items 1 to 31, wherein "r" is selected from 0 to 3, preferably "r" is 0 ("r" is absent).
33. The compound of any one of items 1-32, wherein the compound is selected from 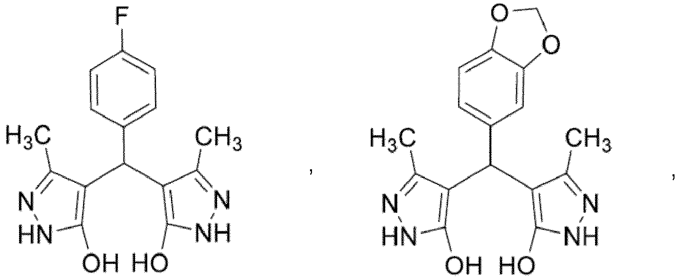 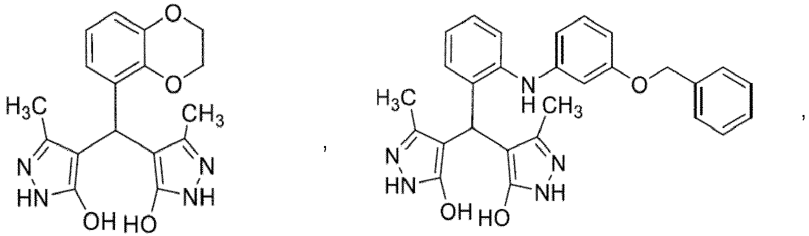 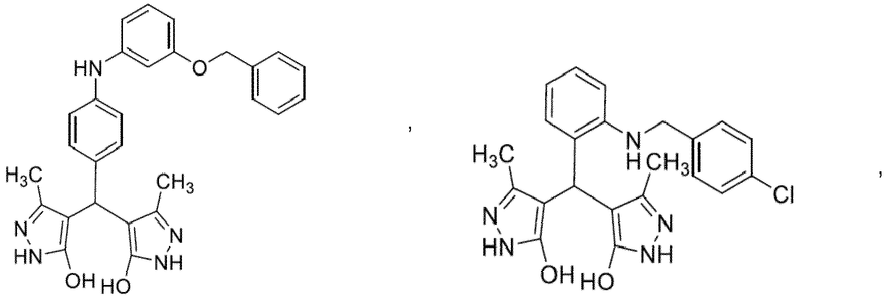 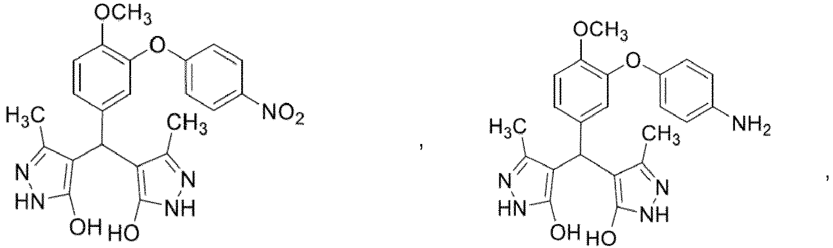 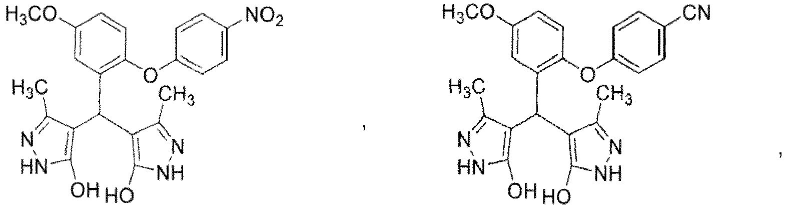 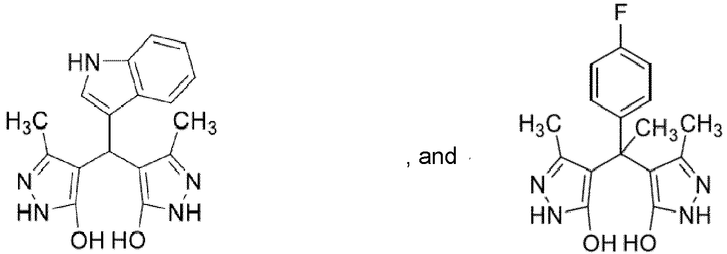
34. An intermediate compound in the synthesis of compounds of any one of items 1 to 33, wherein the intermediate compound is selected from
   a) branched,cyclo or linear (C₁-C₆)-alkyl substituted with CHO;
   b) heterocycle substituted with CHO;
   c) (C₅-C₈)-cycloalkenyl substituted with CHO;
   d) (C₆-C₁₀)-aryl substituted with CHO;
   e) (C₅-C₈)-cycloalkenyl, or the (C₆-C₁₀)-aryl are substituted with CHO and with 1 or 2 groups selected from
      i) OCH₃;
      ii) halogen;
      iii) a substituent having formula II 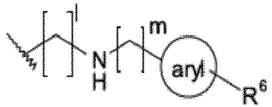 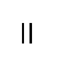 wherein "I" and "m" are independently selected from 0 to 6, the aryl group has 5 to 10 atoms, and wherein R⁶ is H, branched, cyclo or linear (C₁-C₆)-alkyl, or halogen;
      iv) a substituent having formula III 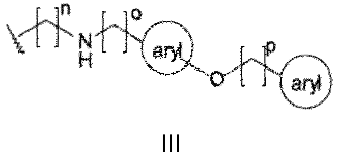 wherein the aryl groups independently have 5 to 10 atoms, and "n", "o" and "p" are independently selected from 0 to 6; and/or
      v) a substituent having formula IV 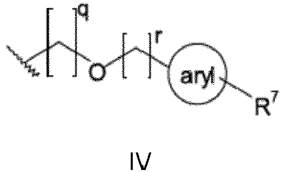 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.
35. The intermediate compound of item 34, wherein the substituted or unsubstituted (C₅-C₈)-cycloalkenyl is selected from cyclopentenyl, cyclopentadienyl, cyclohexenyl, 1,3-cyclohexadiene, cycloheptenyl, 1,3-cycloheptadienyl, cycloheptatrienyl, cyclooctenyl, 1,5-cyclooctadienyl, or cyclooctatetraenyl.
36. The intermediate compound of item 34 or 35, wherein the substituted or unsubstituted (C₆-C₁₀)-aryl is selected from substituted or unsubstituted phenyl, substituted or unsubstituted tolyl, substituted or unsubstituted xylyl, and substituted or unsubstituted naphthyl, preferably substituted phenyl.
37. The intermediate compound of any one of items 34 to 36, wherein the aryl group having 5 to 10 atoms in formulae (II), (III) and/or (IV) is selected from phenyl, pyrrolyl, furanyl, thiophenyl, pyridyl, pyrimidyl, quinolinyl and/or isoquinolinyl, preferably phenyl.
38. The intermediate compound of any one of items 34 to 37, wherein the halogen is selected from F, Cl, Br, and I, preferably F, Cl or I.
39. The intermediate compound of any one of items 34 to 38, wherein the (C₆-C₁₀)-aryl or substituted (C₅-C₈)-cycloalkenyl is substituted with OCH₃ and a substituent having formula IV 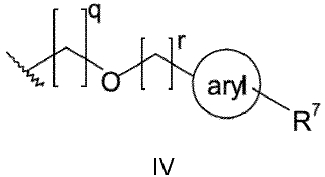 wherein "q" and "r" are independently selected from 0 to 6, the aryl group has 5 to 10 atoms, and wherein R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.
40. The intermediate compound of any one of items 34 to 39, wherein R⁶ in formula (II) and/or R⁷ in formula (IV) are located at position C2, C3 or C4 of the aryl.
41. The intermediate compound of any one of items 34 to 40, wherein the O in formula (III) is located at position C2, C3 or C4 of the aryl or equivalent position.
42. The intermediate compound of any one of items 34 to 41 wherein the substituted (C₆-C₁₀)-aryl or substituted (C₅-C₈)-cycloalkenyl is substituted at positions *ortho, meta* and/or *para* or equivalent positions.
43. The intermediate compound of any one of items 34 to 42 wherein the substituted (C₆-C₁₀)-aryl or substituted (C₅-C₈)-cycloalkenyl is substituted at positions *ortho, meta* and/or *para* or equivalent positions.
44. The intermediate compound of any one of items 34 to 43, wherein "I" is selected from 0 to 3, preferably "I" is 0 ("I" is absent).
45. The intermediate compound of any one of items 34 to 44, wherein "m" is selected from 0 to 3, preferably "m" is 1.
46. The intermediate compound of any one of items 34 to 45, wherein "n" is selected from 0 to 3, preferably "n" is 0 ("n" is absent).
47. The intermediate compound of any one of items 34 to 46, wherein "o" is selected from 0 to 3, preferably "o" is 0 ("o" is absent).
48. The intermediate compound of any one of items 34 to 47, wherein "p" is selected from 0 to 3, preferably "p" is 1.
49. The intermediate compound of any one of items 34 to 48, wherein "q" is selected from 0 to 3, preferably "q" is 0 ("q" is absent).
50. The intermediate compound of any one of items 34 to 49, wherein "r" is selected from 0 to 3, preferably "r" is 0 ("r" is absent).
51. The intermediate compound of any one of items 34 to 50, wherein the intermediate compound is selected from heterocycle substituted with CHO, 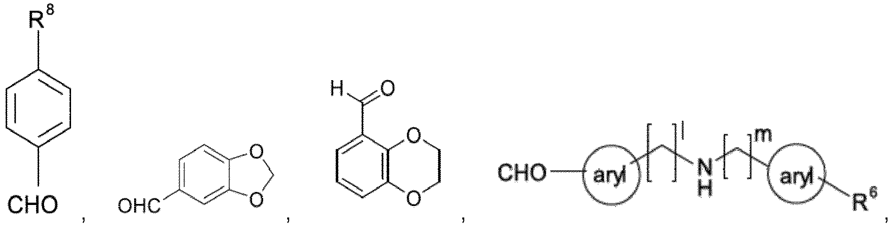 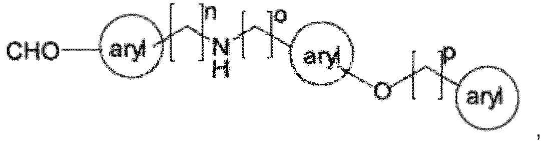 and 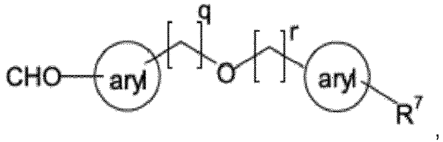
   wherein "I", "m", "n", "o", "q" and "r" are independently selected from 0 to 6, the aryl group has 5 to 10 atoms,
   R⁶ is H, branched, cyclo or linear (C₁-C₆)-alkyl, or halogen; and
   R⁷is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.
52. The intermediate compound of item 34 or 51, wherein the intermediate compound is selected from 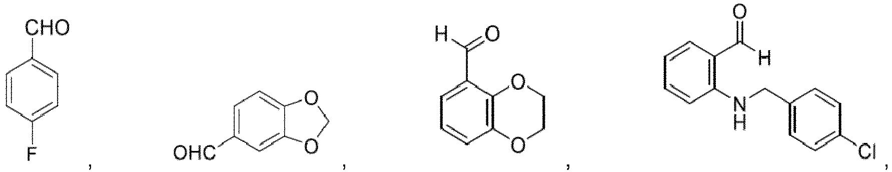 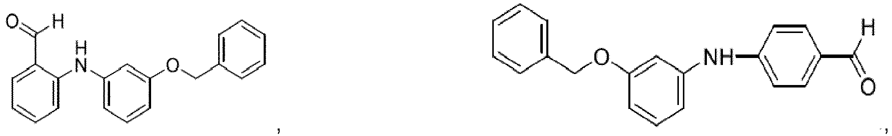 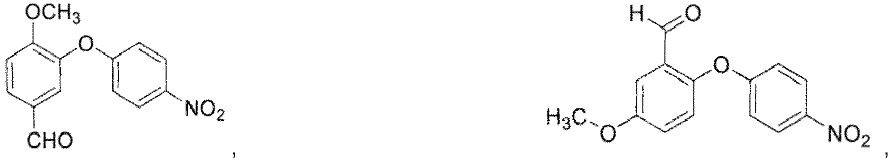 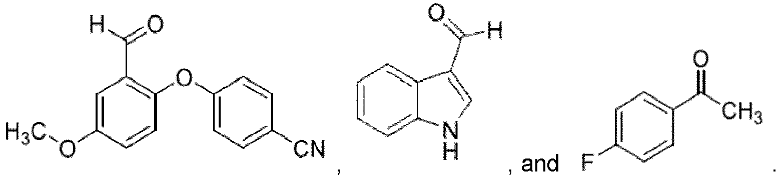
53. The compound as defined in any one of items 1-33 or a salt or solvate thereof or a compound of formula 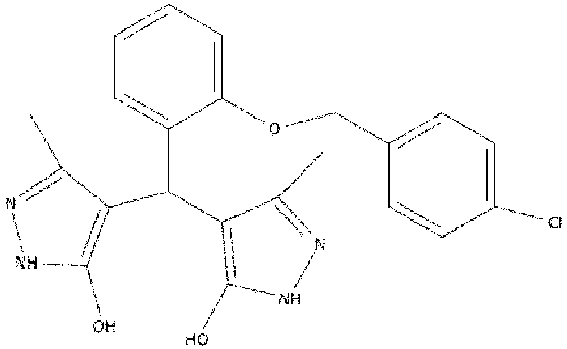 or a salt or solvate thereof for use as a medicament.
54. The compound as defined in any one of items 1-34 or a salt or solvate thereof or a compound of formula 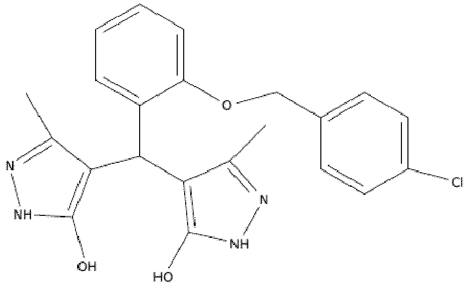 or a salt or solvate thereof
   for use in treating or preventing pancreatic cancer, lung cancer, colorectal cancer, thyroid cancer, testicle cancer, melanoma, bladder cancer, liver cancer, kidney cancer, myelodysplastic syndrome, or leukemia.
55. A pharmaceutical composition comprising the compound as defined in any one of items 1-33 or a pharmaceutically acceptable salt or solvate thereof or a compound of formula 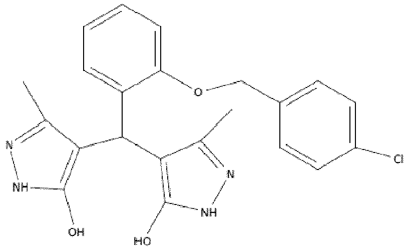 or salt or solvate thereof.
56. The pharmaceutical composition of item 55 for use as a medicament.
57. The pharmaceutical composition of item 55 or 56 for use in treating pancreatic cancer, lung cancer, colorectal cancer, thyroid cancer, testicle cancer, melanoma, bladder cancer, liver cancer, kidney cancer, myelodysplastic syndrome, or leukemia.
58. The pharmaceutical composition of item 55 or the pharmaceutical composition for use according to item 56 or 57, wherein the pharmaceutical composition further comprises an excipient, carrier or solvent.
59. The compound of any one of items 1-33, the compound for use according to item 53 or 54, the pharmaceutical composition of item 55 or 58, or the pharmaceutical composition for use according to any one of items 56 to 58, wherein the compound increases RAS signaling.
60. The compound of any one of items 1-33 or 59, the compound for use according to item 53, 54, or 59 the pharmaceutical composition of item 55, 58 or 59, or the pharmaceutical composition for use according to any one of items 56 to 59, wherein the compound increases RAS signaling compared to a control.
61. The compound of any one of items 1-33, 59, or 60, the compound for use according to item 53, 54, 59 or 60 the pharmaceutical composition of item 55, 58 to 60, or the pharmaceutical composition for use according to any one of items 56 to 60, wherein the compound's increase in RAS signaling is detected by
   a) seeding DLD-1 KRAS ^{WT/G13D} clone V15 endogenously expressing oncogenic KRAS or DLD-1 KO KRAS ^{WT/+} clone DWT7 cells stably expressing HA-KRAS G12V in media comprising 10 % FBS for 24 hours;
   b) serum starving the cells are 24 hours;
   c) incubating the cells for 3 hours with 100 µM of the compound, for 30 minutes with 10 % FBS as positive control and 0 % FBS as negative control;
   d) lysing the cells in a buffer containing 67 mM Tris-HCl and 2 % SDS having a pH of 6.8 and then incubating at 97°C for 15 minutes;
   e) determining the protein concentration of the lysates using the Lowry method;
   f) resolving 15 µg of protein on SDS-PAGE
   g) transferring the separated proteins onto a PVDF membrane;
   h) detecting activation of Ras effector proteins ERK and AKT using anti-pAKT or anti-pERK antibodies;
   i) comparing the signal obtained for the compound to the signal obtained for the 0 % FBS negative control and 10% FBS as positive control;
   j) detecting an increased signal for the compound of p-AKT and p-ERK compared to the negative control.
62. The compound of any one of items 1-33, 59 to 61, the compound for use according to item 53, 54, 59 to 61 the pharmaceutical composition of item 55, 58 to 61, or the pharmaceutical composition for use according to any one of items 56 to 61, wherein the compound decreases tumor cell viability.
63. The compound of any one of items 1-33, 59 to 62, the compound for use according to item 53, 54, 59 to 62 the pharmaceutical composition of item 55, 58 to 62, or the pharmaceutical composition for use according to any one of items 56 to 62, wherein the compound decreases tumor cell viability compared to a control.
64. The compound of any one of items 1-33, 59 to 63, the compound for use according to item 53, 54, 59 to 63 the pharmaceutical composition of item 55, 58 to 63, or the pharmaceutical composition for use according to any one of items 56 to 63, wherein the compound's decrease in tumor cell viability is detected by
   a) culturing 10000 DLD-1 KRAS^{WT/G13D} clone V15 cells expressing endogenous oncogenic KRAS in 50 µl of a medium comprising 10 % FBS in a 96-well plate for 24 hours;
   b) adding 50 µl of the compound or 50 µl of medium as negative control;
   c) performing an MTS viability assay;
   d) calculating the percentage of viability by dividing the absorbance of each well by the average absorbance of the negative control wells;
   e) detecting a decreased viability of the cells compared to control cells not treated with the compound.
65. The compound of any one of items 1-33, 59 to 64, the compound for use according to item 53, 54, 59 to 64 the pharmaceutical composition of item 55, 58 to 64, or the pharmaceutical composition for use according to any one of items 56 to 64, wherein the compound interacts with Glu168 and Lys169 of KRAS of SEQ ID NO. 1.
66. The compound of any one of items 1-33, 59 to 65, the compound for use according to item 53, 54, 59 to 65 the pharmaceutical composition of item 55, 58 to 65, or the pharmaceutical composition for use according to any one of items 56 to 65, wherein the compound interacts with Glu168 and Lys169 of KRAS represented by SEQ ID No. 1.
67. The compound of any one of items 1-33, 59 to 66, the compound for use according to item 53, 54, 59 to 66 the pharmaceutical composition of item 55, 58 to 66, or the pharmaceutical composition for use according to any one of items 56 to 66, wherein the compound interacts with oncogenic KRAS -GST-KRAS-G12V-corresponding to amino acid 1 to 160 of KRAS as represented by SEQ ID No. 2.
68. The compound of any one of items 1-33, 59 to 67, the compound for use according to item 53, 54, 59 to 67 the pharmaceutical composition of item 55, 58 to 67, or the pharmaceutical composition for use according to any one of items 56 to 67, wherein the interaction of the compound with GST-KRAS-G12V is detected via plasmon resonance analysis.
69. The compound of any one of items 1-33, 59 to 68, the compound for use according to item 53, 54, 59 to 68 the pharmaceutical composition of item 55, 58 to 68, or the pharmaceutical composition for use according to any one of items 56 to 68, wherein the compound competes with Calmodulin -CaM- represented by SEQ ID No. 3 for binding to oncogenic GST-KRAS-G12V.
70. The compound of any one of items 1-33, 59 to 69, the compound for use according to item 53, 54, 59 to 69 the pharmaceutical composition of item 55, 58 to 69, or the pharmaceutical composition for use according to any one of items 56 to 69, wherein the competing is detected by CaM-sepharose pulldown assay using GST-KRAS-G12V in the presence of the compound.
71. The compound of any one of items 1-33, 59 to 70, the compound for use according to item 53, 54, 59 to 70 the pharmaceutical composition of item 55, 58 to 70, or the pharmaceutical composition for use according to any one of items 56 to 70, wherein the compound increases HA-KRAS G12V interaction with BRAF or P-C-RAF S338 compared to a control.
72. The compound of any one of items 1-33, 59 to 71, the compound for use according to item 53, 54, 59 to 71 the pharmaceutical composition of item 55, 58 to 71, or the pharmaceutical composition for use according to any one of items 56 to 71, wherein the compound increases RAS signaling of cells expressing KRAS G12V compared to control
73. The compound of any one of items 1-33, 59 to 72, the compound for use according to item 53, 54, 59 to 72 the pharmaceutical composition of item 55, 58 to 72, or the pharmaceutical composition for use according to any one of items 56 to 72, wherein the compound reduces the cell viability of DLD-1 cells to a larger extend than erlotinib, wherein the compound and erlotinib are used in the same concentration.
74. A method for the preparation of a compound as defined in any one of items 1-33 or the pharmaceutical composition of item 55, the process comprising contacting 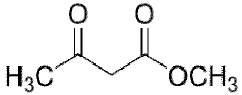 NH₂-NH₂·H₂O with and with one intermediate compound, wherein the intermediate compound defined in any one of items 34-52.
75. A kit or package comprising the compound as defined in any one of items 1-33, or the pharmaceutical composition of item 55.
76. The kit or package of item 75, wherein the kit or package further comprises instructions for the medical use of the compound or of the pharmaceutical composition as defined in any one of items 53, 54, 56 or 57 and/or a unit dosage form of the compound as defined in any one of items 1-33, or of the pharmaceutical composition of item 55.
77. Use of a compound or a salt or solvate thereof as defined in any one of items 1-33, or the pharmaceutical composition of item 55, for the preparation of a medicament.
78. Use of a compound or a salt or solvate thereof as defined in items 1-33 or the pharmaceutical composition as defined in item 55 for the preparation of a medicament for treating pancreatic cancer, lung cancer, colorectal cancer, thyroid cancer, testicle cancer, melanoma, bladder cancer, liver cancer, kidney cancer, myelodysplastic syndrome, or leukemia.
79. A method of treating a disease, the method comprising administering to a subject in need thereof a therapeutically effective amount of a compound as defined in any one of items 1-33 or a salt or solvate thereof, or a pharmaceutical composition as defined in item 55.
80. A method of treating pancreatic cancer, lung cancer, colorectal cancer, thyroid cancer, testicle cancer, melanoma, bladder cancer, liver cancer, kidney cancer, myelodysplastic syndrome, or leukemia, the method comprising administering to a subject in need thereof a therapeutically effective amount of a compound as defined in any one of items 1-33 or a salt or solvate thereof, or a pharmaceutical composition as defined in item 55.

### Sequences referred to herein

| | |
|---|---|
| SEQ ID NO. 1 | |
| Uniprot no. P01116-2 (Name Isoform 2B of KRAS) version 1 of 1986-07-21 | |
| SEQ ID No. 2 | MTEYKLVVVGAVGVGKSALTIQLIQNHFVDEYDPTIE DSYRKQVVIDGETCLLDILDTAGQEEYSAMRDQYMR TGEGFLCVFAINNTKSFEDIHHYREQIKRVKDSEDVP MVLVGNKCDLPSRTVDTKQAQDLARSYGIPFIETSA KTRQGVDDAFYTLVREIRKHKEKMSKDGKKKKKK SKTKCVIM |
| Uniprot no. P01116-2 (Name Isoform 2B of KRAS) version 1 of 1986-07-21 including G12V amino acid substitution | |
| SEQ ID NO. 3 | |
| Uniprot no. P0DP23 . CALM1_HUMAN (Uniport) version 1 of 2017-05-10 | |

### Examples

### Example 1. Preparation of 4,4'-((4-fluorophenyl)methylene)-bis(3-methyl-1H-pyrazol-5-ole) (1)

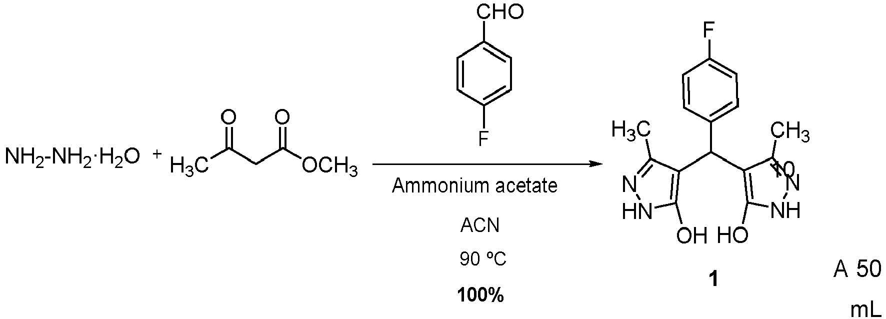

round-bottomed flask equipped with a magnetic stirring bar and cooled down to 0 ± 10 °C with an ice-bath, was charged with hydrazine (d = 1.03 g/mL, 0.5 mL, 10 mmol, 2 eq), methyl acetoacetate (1.07 mL, 10 mmol, 2 eq), 4-fluorobenzaldehyde (0.53 mL, 5 mmol, 1 eq) and finally with ammonium acetate (0.77 g, 10 mmol, 2 eq). All compounds were dissolved in 5 mL of ACN. The resulting mixture was closed with a reflux condenser and the system was heated at 90 ± 10 °C under constant stirring for 24 hours. During the reaction time a spontaneous solid was formed, which was filtered under reduced pressure. The solid formatted was dried and ¹H spectrum of the final compound was carried out. No purification by column chromatography was performed since the product was obtained with enough purity as shown by TLC and ¹H spectrum. A white solid was obtained.
*Yield:* 100%

### Analytical data

- R_{f}: 0.125 (Hexane/Ethyl Acetate (4:6))
- Melting point: 244-246 °C (Methanol)
- Aspect: white solid
- ¹H NMR (DMSO-d₆, 400 MHz) δ(ppm), 2.01 (s, 6H, CH₃- (x2)); 4.72 (s, 1H, Ar-CH-); 6.99 (dt, *J*₁ = 2, *J*₂ = 8.8 Hz, 2H, H-2', H-6'); 7.12 (dt, *J*₁ = 2, *J*₂ = 8.8 Hz, 2H, H-3', H-5').
- ¹³C NMR (DMSO-d₆, 100.6 MHz) δ(ppm), 10.8 (CH₃-Ar- (x2)); 32.5 (CH, Ar-CH); 104.6 (C, C-4 (x2)); 114.6 (CH, d, *J =* 20 Hz, C-3', C-5'); 129.6 (CH, d, *J =* 7.5 Hz, C-2', C-6'); 139.8 (C, C-3); 140.1 (C, C-3); 159.6 (C, d, *J =* 242 Hz, C-4'); 161.4 (C, C-5 (x2)).

### Example 2. Preparation of 4,4'-(benzo[d][1,3]dioxole-5-ylmethylene)-bis(3-methyl-1H-pyrazol-5-ole) (2)

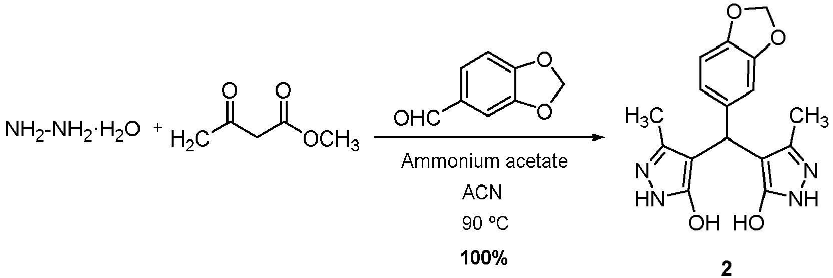

A 50 mL round-bottomed flask equipped with a magnetic stirring bar and cooled down to 0 ± 10 °C with an ice-bath, was charged with hydrazine (d = 1.03 g/mL, 0.5 mL, 10 mmol, 2 eq), methyl acetoacetate (1.07 mL, 10 mmol, 2 eq), benzo[d][1,3]dioxole-5-carbaldehyde (0.751 g, 5 mmol, 1 eq) and finally with ammonium acetate (0.771 g, 10 mmol, 2 eq). All compounds were dissolved in 5 mL of ACN. The resulting mixture was closed with a reflux condenser and the system was heated at 90 ± 10 °C under constant stirring for 24 hours. The solvent was evaporated under vacuum. Then, ¹H spectrum of the residue was carried out in order to identify the desired product. No purification by column chromatography was performed since the product was obtained with enough purity (100%) as shown by TLC and ¹H spectrum. An orange solid was obtained.
*Yield:* 100%

### Analytical data

- R_{f}: 0.128 (Hexane/Ethyl Acetate (1:1))
- Melting point: 251-253 °C (Methanol)
- Aspect: orange solid
- ¹H NMR (DMSO-d₆, 400 MHz) δ(ppm), 2.04 (s, 6.4, CH₃ (x2)); 4.64 (s, 1H, Ar-CH-); 5.98 (s, 2H, O-CH₂-O-); 6.60 (d, *J=* 7 Hz, 1H, H-6'); 6.67 (s, 1H, H-4'); 6.70 (d, *J =* 7 Hz, 1H, H-7').
- ¹³C NMR (DMSO-d₆, 100.6 MHz) δ(ppm), 10.9 (CH₃ (x2)); 39.2 (CH, Ar-CH-); 100.9 (CH₂, O-CH₂-O-); 104.5 (C, C-4 (x2)); 107.9 (CH, C-7'); 108.6 (CH, C-4'); 120.6 (CH, C-6'); 138.5 (C, C-5'); 140.3 (C, C-3 (x2)); 145.3 (C, C-7'a); 147.3 (C, C-3'a); 161.2 (C, C-5); 173.2 (C, C-5 *bis).*

### Example 3. Preparation of 4,4'-((2,3-dihydrobenzo[b][1,4]dioxin-5-yl)methylene)-bis(3-methyl-1H-pyrazol-5-ole) (3)

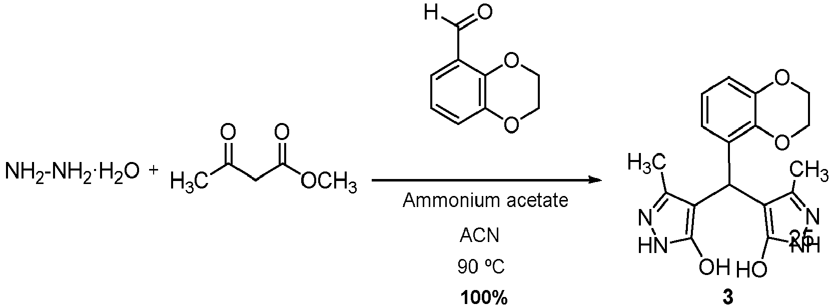

A 50 mL round-bottomed flask equipped with a magnetic stirring bar and cooled down to 0 ± 10 °C with an ice-bath, was charged with hydrazine (d = 1.03 g/mL, 0.06 mL, 2.43 mmol, 2 eq), methyl acetoacetate (0.14 mL, 2.43 mmol, 2 eq), 2,3-dihydrobenzo[*b*][1,4]dioxin-5-carbaldehyde (0.2g mL, 1.22 mmol, 1 eq) and finally with ammonium acetate (0.096 g, 2.43 mmol, 2 eq). All compounds were dissolved in 5 mL of ACN. The resulting mixture was closed with a reflux condenser and the system was heated at 90 ± 10 °C under constant stirring for 24 hours. During the reaction time a spontaneous solid was formed, which was filtered under reduced pressure. The solid formatted was dried and ¹H spectrum of the final product was carried out. No purification by column chromatography was performed since the product was obtained with enough purity as shown by TLC and ¹H spectrum. A yellowish solid was obtained.
*Yield:* 100%

### Analytical data

- R_{f}: 0.04 (Hexane/Ethyl Acetate (5:5))
- Melting point: 205-210 °C (Methanol)
- Aspect: yellowish solid
- ¹H NMR (DMSO-d₆, 400 MHz) δ(ppm), 2.00 (s, 6H, CH₃ (x2)); 4.14 (d, *J =* 8 Hz, 4H, CH₂-O- x2); 4.99 (s, 1H, CH-); 6.60 - 6.62 (m, 2H, H-7', H-8'); 7.12 (d, *J =* 8 Hz, 1H, H-6').
- ¹³C NMR (DMSO-d₆, 100.6 MHz) δ(ppm), 9.4 (CH₃ (x2)); 25.15 (CH, Ar-CH); 62.6 - 62.9 (CH₂, CH₂-O- ); 87.8 (CH, C-4' (x2)); 102.6 (C, C-4 (x2)); 113.4 (CH, C-8'); 118.6 (CH, C-7'); 120.2 (CH, C-6'); 131.5 (C, C-3 (x2)); 138.7 (C, C-3 (x2)); 139.2 (C, C-8'a); 141.7 (C, C-4'a); 160.1 (C, C-5 (x2)); 175.5 (C, C=O).

### Example 4. Preparation of 4,4'-((2-((4-chlorobenzyl)amino)phenyl)methylene)-bis(3-methyl-1H-pyrazol-5-ole) (4)

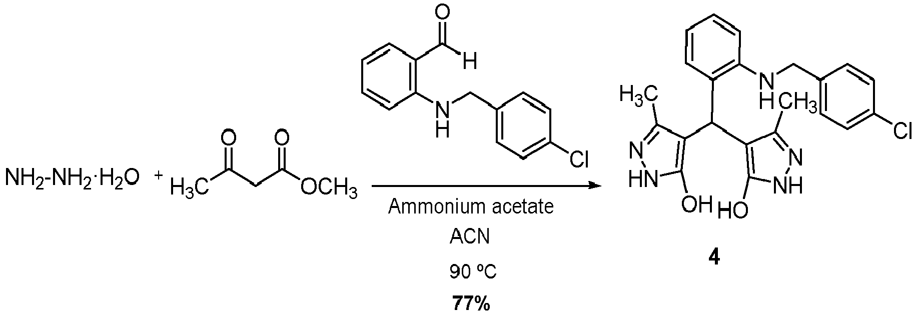

A 50 mL round-bottomed flask equipped with a magnetic stirring bar and cooled down to 0 ± 10 °C with an ice-bath, was charged with hydrazine (d = 1.03 g/mL, 0.06 mL, 1.25 mmol, 2 eq), methyl acetoacetate (0.14 mL, 1.25 mmol, 2 eq), the aldehyde (0.154 g mL, 0.63 mmol, 1 eq) and finally with ammonium acetate (0.096 g, 1.25 mmol, 2 eq). All compounds were dissolved in 5 mL of ACN. The resulting mixture was closed with reflux condenser and the system was heated at 90 ± 10 °C under constant stirring for 24 hours. During the reaction time a spontaneous solid was formed, which was filtered under reduced pressure. The solid formatted was dried and ¹H spectrum of the obtained residue was carried out. No purification by column chromatography was performed since the product was obtained with enough purity as shown by TLC and ¹H spectrum. A yellowish solid was obtained.
*Yield:* 77%

### Analytical data

- R_{f}: 0.05 (Hexane/Ethyl Acetate (5:5))
- Melting point: 185-188 °C (Methanol)
- Aspect: yellowish solid
- ¹H NMR (CDCl₃, 400 MHz) δ(ppm), 1.99 (s, 6H, CH₃ (x2)); 4.32 (s, 1H, Ar-CH-); 4.42 (s, 2H, CH₂-Ar); 6.55 (d, *J=* 8 Hz, 2H, H-2", H-6"); 6.60 (d, *J=* 8 Hz, 2H, H-3", H-5"); 7.33-7.34 (m, 4H, H-3', H-4', H-5', H-6'); 8.51 (s, 1H, NH).

### Example 5. Preparation of 4,4'-((2-((3-(benzyloxy)phenyl) amino)phenyl)methylene)-bis(3-methyl-1H-pyrazol-5-ole) (5)

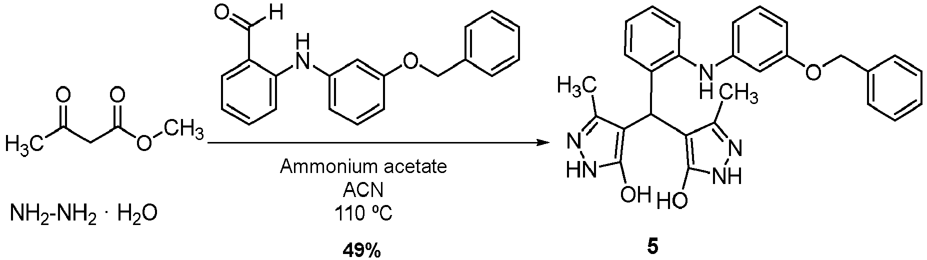

A 50 mL round-bottomed flask equipped with a magnetic stirring bar and cooled down to 0 ± 10 °C with an ice-bath, was charged with hydrazine (d = 1.03 g/mL, 0.048 mL, 0.98 mmol, 2 eq), methyl acetoacetate (0.11 mL, 0.98 mmol, 2 eq), the initial aldehyde 97 (0.150 g, 0.49 mmol, 1 eq) and finally with ammonium acetate (0.076 g, 0.98 mmol, 2 eq). All compounds were dissolved in 5 mL of ACN. The resulting mixture was closed with a reflux condenser and the system was heated at 110 ± 10 °C under constant stirring for 24 hours.

The solvent was removed under pressure. Then, the crude product was crystallized with ethyl acetate. The solid formed was filtered and dried under pressure. Finally, ¹H spectrum of the obtained residue was carried out. No purification by column chromatography was performed since the product was obtained with enough purity as shown by TLC and ¹H spectrum. A brown solid was obtained.
*Yield:* 49%

### Analytical data

- R_{f}: 0.08 (Hexane/Ethyl Acetate (3:7))
- Melting point: 150-155 °C (Methanol)
- Aspect: brown solid
- ¹H NMR (CDCl₃, 400 MHz) δ(ppm), 2.10 (s, 6H, CH₃ (x2)); 4.8 (s, 2H, CH₂-O-); 5.72 (s, 1H, Ar-CH-); 6.85 (d, *J=* 9 Hz, 2H, H-3', H-6'); 6.85-6.87 (m, 1H, H-4‴); 6.92-6.95 (m, 2H, H-4', H-5'); 7.02 (t, *J=* 7.6 Hz, 2H, H-3‴, H-5‴); 7.08-7.10 (m, 2H, H-4", H-5"); 7.16 (d, *J* = 7 Hz, 1H, H-6"); 7.19 (d, *J* = 2 Hz, 1H, H-2"); 7.54 (d, *J=* 7.7 Hz, 1H, H-2‴)*; 7.60 (d, *J=* 7.7 Hz, 1H, H-6‴)*; 8.50 (bs, 2H, OH). *Interchangeable protons
- ¹³C NMR (CDCl₃, 100.6 MHz) δ(ppm), 10.2 (CH₃ (x2)); 30.0 (CH, Ar-CH-); 52.4 (CH₂, CH₂-O-); 101.3 (C, C-4 (x2)); 108.4 (CH, C-2"); 115.0 (CH, C-6"); 115.2 (CH, C-6'); 115.4 (CH, C-5'); 124.1 (CH, C-3'); 124.3 (CH, C-4'); 125.3 (CH, C-4"); 127.5 (C, C-1'); 128.0 (CH, C-2‴, C-6‴); 128.1 (CH, C-4‴); 128.2 (CH, C-3‴, C-5‴); 129.0 (CH, C-5"); 136.8 (C, C-1‴); 140.1 (C, C-3 (x2)); 151,4 (C, C-1"); 158.4 (C, C-2'); 162.0 (C, C-3"); 168.4 (C, C-5); 168.7 (C, C-5).
- HRMS ESI (+) m/z: calculated mass for C₂₈H₂₈N₅O₃ 482.2114, found: 482.2130.

### Example 6. Preparation of 4,4'-((4-((3-(benzyloxy)phenyl) amino)phenyl)methylene)-bis(3-methyl-1H-pyrazol-5-ole) (6)

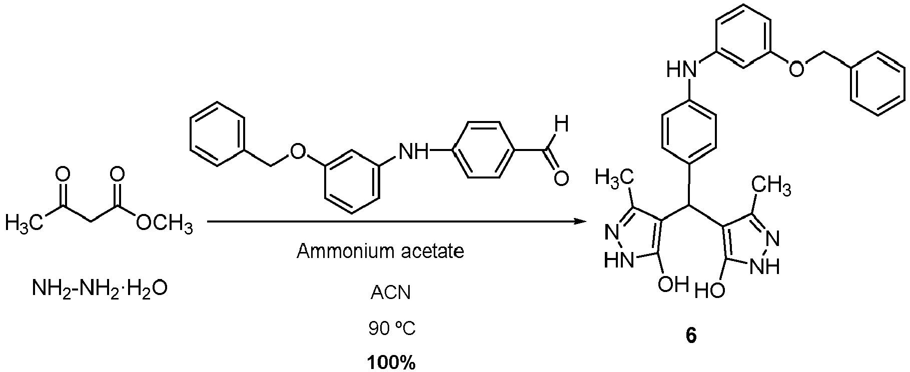

A 50 mL round-bottomed flask equipped with a magnetic stirring bar and cooled down to 0 ± 10 °C with an ice-bath, was charged with hydrazine (d = 1.03 g/mL, 0.058 mL, 1.19 mmol, 2 eq), methyl acetoacetate (0.128 mL, 1.19 mmol, 2 eq), 4-((3-(benzyloxy)phenyl)amino)benzaldehyde (0.181 g, 0.597 mmol, 1 eq) and finally with ammonium acetate (0.092 g, 1.19 mmol, 2 eq). All components were dissolved in 5 mL of ACN. The resulting mixture was closed with a reflux condenser and the system was heated at 90 ± 10 °C under constant stirring for 24 hours. The solvent was evaporated under vacuum. Then, ¹H spectrum of the obtained residue was carried out in order to identify the desired product. No purification by column chromatography was performed since the product was obtained with enough purity (100%) as shown by TLC and ¹H spectrum. An orange-red solid was obtained.
*Yield:* 100%

### Analytical data

- R_{f}: 0.11 (Hexane/Ethyl Acetate (1:1))
- Melting point: 180-183 °C (Methanol)
- Aspect: orange-red solid
- ¹H NMR (DMSO-d₆, 400 MHz) δ(ppm), 2.13 (s, 6H, CH₃ (x2)); 5.01 (s, 1H, CH-); 5.19 (s, 2H, CH₂-O-); 6.35 (dd, *J*₁ = 2, *J*₂ = 8 Hz, 1H, H-4"); 6.61 (d, *J=* 8, 1H, H-6"); 6.65 (d, *J =* 2 Hz; H-2"); 6.94 (d, *J =* 8.5 Hz, 2H, H-2', H-6'); 7.04 (d, *J =* 8.5 Hz, 2H, H-3', H-5'); 7.02 (t, *J =* 8 Hz, 1H, H-5"); 7.26 (t, *J =* 7. 12 Hz, 1H, H-4‴); 7.35 (t, *J =* 7.12 Hz, 2H, H-3‴, H-5‴); 7.43 (d, *J=* 7.12 Hz, 2H, H-2‴, H-6‴); 7.98 (bs, 1H, NH); 8.3 (bs, 2H, OH).
- ¹³C NMR (DMSO-d₆, 100.6 MHz) δ(ppm), 11.6 (CH₃ (x2)); 32.6 (CH, Ar-CH-); 69.4 (CH₂-O-); 89.3 (CH, C-3', C-5'); 102.3 (CH, C-2"); 104.8 (CH, C-4"); 105.7 (C, C-4 (x2)); 109.0 (CH, C-6"); 117.9 (CH, C-4‴); 127.9 (CH, C-2‴, C-6‴); 128.1 (CH, C-3‴, C-5‴); 128.8 (CH, C-2', C-6'); 130.2 (CH, C-5"); 135.7 (C, C-1'); 137.7 (C, C-1‴); 139.9 (C, C-3 (x2)); 140.1 (C, C-4'); 159.6 (C, C-1"); 161.5 (C, C-3"); 172.7 (C, C-5 (x2)).

### Example 7. Preparation of 4,4'-((4-methoxy-3-(4-nitrophenoxy)phenyl)methylene)-bis(3-methyl-1H-pyrazol-5-ole) (7)

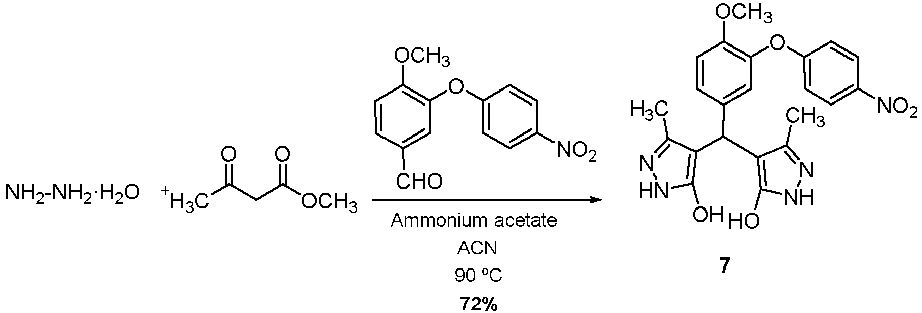

A 50 mL round-bottomed flask equipped with a magnetic stirring bar and cooled down to 0 ± 10 °C with an ice-bath, was charged with hydrazine (d = 1.03 g/mL, 0.23 mL, 4.81 mmol, 2 eq), methyl acetoacetate (0.52 mL, 4.81 mmol, 2 eq), 4-methoxy-3-(4-nitrophenoxy)benzaldehyde (0.785 mL, 2.40 mmol, 1 eq) and finally with ammonium acetate (0.37 g, 4.81 mmol, 2 eq). All components were dissolved in 5 mL of ACN. The resulting mixture was closed with a reflux condenser and the system was heated at 90 ± 10 °C under stirring for 24 hours. The solvent was evaporated under vacuum. Then, ¹H spectrum was carried out in order to identify the desired product. The crude reaction was purified by column chromatography on silica gel eluting with mixtures of hexane/ethyl acetate and methanol with increasing polarity. The desired product eluted with a polarity of ethyl acetate/methanol (80:20). The desired product was a brown-orange solid.
*Yield:* 72%

### Analytical data

- R_{f}: 0.78 (Methanol)
- Melting point: 201-203 °C (methanol)
- Aspect: brown-orange solid
- ¹H NMR (DMSO-d₆, 400 MHz) δ(ppm), 2.1 (s, 6H, CH₃ (x2)); 3.67 (s, 3H, CH₃-O-); 4.48 (s, 1H, CH-Ar); 6.89 (d, *J* = 2 Hz, 1H, H-2'); 6.93 (d, *J* = 9 Hz, 2H, H-2", H-6"); 7.01 (d, *J =* 8.6 Hz, 1H, H-5'); 7.03 (dd, *J=* 2, *J =* 8.6 Hz, 1H, H-6'); 8.18 (d, *J=* 9 Hz, 2H, H-3", H-5").
- ¹³C NMR (DMSO-d₆, 100.6 MHz) δ(ppm), 10.7 (CH₃ (x2)); 32.4 (CH, Ar-CH-); 56.2 (CH₃-O-); 104.5 (C, C-4 (x2)); 113.4 (CH, C-5'); 115.9 (CH, C-2", C-6"); 122.0 (CH, C-6'); 126.2 (CH, C-2'); 126.4 (CH, C-3", C-5"); 137.2 (C, C-3 (x2)); 141.2 (C, C-4"); 142.1 (C, C-3'); 149.4 (C, C-4'); 162.9 (C, C-5); 163.6 (C, C-1").
- HMRS ESI (+) m/z: calculated mass for C₂₂H₂₂N₅O₆ 452.1496, found 452.1500.

### Example 8. Preparation of 4,4'-((3-(4-aminophenoxy)-4-metoxyphenyl)methylene)-bis(3-methyl-1H-pyrazol-5-ole) (8)

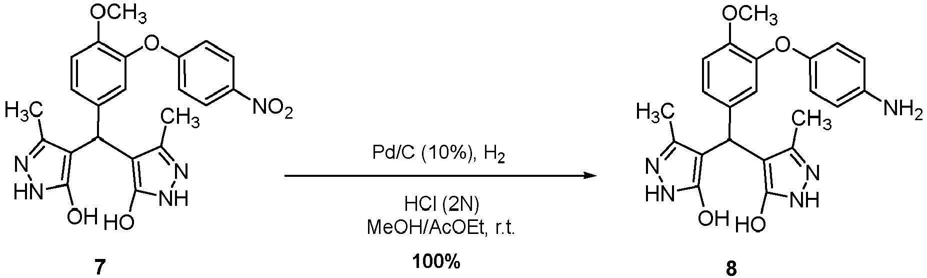

A 50 mL round-bottomed flask for catalytic hydrogenations was charged with compound (0.091 g, 0.20 mmol, 1 eq) dissolved in a mixture of 10 mL of methanol and 5 mL of ethyl acetate. The catalyst was then added Pd-C 10% (10% p/p). Then, three drops of hydrochloric acid (2N) were added to the solution in order to catalyze the reaction. The reaction mixture was stirred at room temperature for 8 days. The theoretical volume required for the reaction was 9 mL. Given that the hydrogenation apparatus was not hermetical and may leak, the consumed volume was 145 mL, higher than expected. The crude reaction was filtered by means of a pleated filter and washed with 20 mL of methanol and was then collected in a round-bottomed flask of 100 mL capacity. Finally, methanol was removed under reduced pressure. Then, ¹H spectrum of the obtained residue was carried out confirming the formation of the expected product. In this case no purification by column chromatography was performed since as shown by ¹H spectrum the product was pure.
*Yield:* 100%

### Analytical data

- R_{f}: 0,05 (Ethyl Acetate/Methanol (8:2))
- Melting point: 160-162 °C (Methanol)
- Aspect: brown solid.
- ¹H NMR (DMSO-d₆, 400 MHz) δ(ppm), 1.99 (s, 6H, CH₃ (x2)); 4.90 (s, 1H, Ar-CH-); 6.30 (dd, *J*₁ = 2, *J*₂ = 8.8 Hz, 1H, H-6'); 6.83 (d, *J=* 8.9 Hz, 2H, H-3", H-5"); 6.97 (d, *J =* 2 Hz, 1H, H-2'); 7.06 (d, *J =* 8.8 Hz, 1H, H-5'); 7.28 (d, *J =* 8.9 Hz, H-2", H-6").

### Example 9. Preparation of 4,4'-((5-methoxy-2-(4-nitrophenoxy)phenyl)methylene)-bis(3-methyl-1H-pyrazol-5-ole) (9)

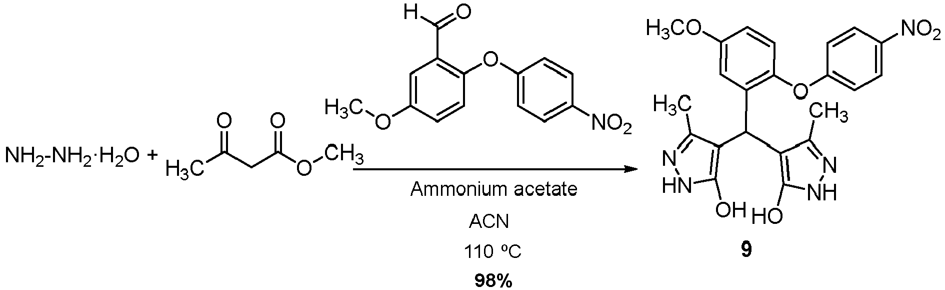

A 50 mL round-bottomed flask equipped with a magnetic stirring bar and cooled down to 0 ± 10 °C with an ice-bath, was charged with hydrazine (d = 1.03 g/mL, 0.036 mL, 0.72 mmol, 2 eq), methyl acetoacetate (0.079 mL, 0.72 mmol, 2 eq), the starting aldehyde (0.100 g, 0.36 mmol, 1 eq) and finally with ammonium acetate (0.056 g, 0.72 mmol, 2 eq). All components were dissolved in 5 mL of ACN. The resulting mixture was closed with a reflux condenser and the system was heated at 110 ± 10 °C under stirring for 24 hours. The solvent was evaporated under vacuum. Then, ¹H spectrum of the obtained residue was carried out in order to identify the expected product. No purification by column chromatography was performed since the product was obtained with enough purity as shown by TLC and ¹H spectrum. A yellow solid was obtained.
*Yield:* 98%

### Analytical data

- R_{f}: 0.04 (Hexane/Ethyl Acetate (3:7))
- Melting point: 200-205 °C (Methanol)
- Aspect: yellow solid
- ¹H NMR (DMSO-d₆, 400 MHz) δ(ppm), 1.92 (s, 6H, CH₃ (x2)); 3.70 (s, 3H, CH₃-O-); 4.80 (s, 1H, Ar-CH-); 6.79-6.81 (m, 1H, H-4'); 6.82 (d, *J=* 9.2 Hz, 2H, H-2", H-6"); 6.89 (d, *J=* 8.7 Hz, 1H, H-6'); 7.21 (d, *J=* 3 Hz, 1H, H-3'); 8.11 (d, *J=* 9.2 Hz, 2H, H-3", H-5").
- HRMS ESI (+) m/z: calculated mass for C₂₂H₂₂N₅O₆ 452.1492, found 452.1623.

### Example 10. Preparation of 4-(2-(bis(5-hydroxy-3-methyl-1H-pyrazol-4-yl)methyl)-4-methoxy phenoxy)benzonitrile (10)

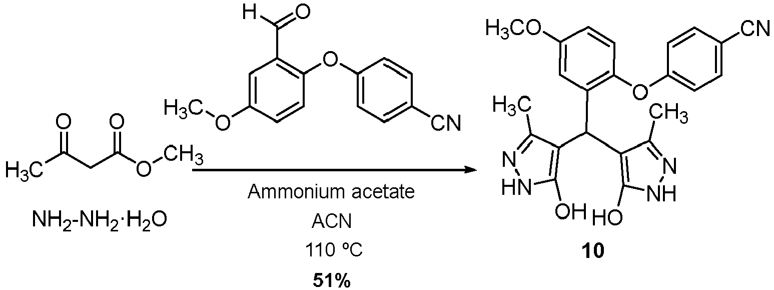

A 50 mL round-bottomed flask equipped with a magnetic stirring bar and cooled down to 0 ± 10 °C with an ice-bath, was charged with hydrazine hydrate (d = 1.03 g/mL, 0.014 mL, 0.292 mmol, 2 eq), methyl acetoacetate (0.03 mL, 0.292 mmol, 2 eq), the initial aldehyde (0.063 g, 0.146 mmol, 1 eq) and finally with ammonium acetate (0.022 g, 1.25 mmol, 2 eq). All components were dissolved in 5 mL of ACN. The resulting mixture was closed with a reflux condenser and the system was heated at 90 ± 10 °C under constant stirring for 24 hours. During the reaction a spontaneous solid was formed, which was filtered under reduced pressure. The solid formatted was dried and ¹H spectrum of the obtained residue was carried out. No purification by column chromatography was performed since the product was obtained with enough purity as shown by TLC and ¹H spectrum. A brown solid was obtained.
*Yield:* 51%

### Analytical data

- R_{f}: 0.42 (Hexane/Ethyl Acetate (8:2))
- Melting point: 203-206 °C (Ethyl Acetate)
- Aspect: brown solid
- ¹H NMR (DMSO-d₆, 400 MHz) δ(ppm), 2.01 (s, 6H, CH₃- (x2)); 5.20 (s, 1H, CH); 5.97 (bs, 1H, H-6'); 6.57 (t, *J =* 8 Hz, 1H, H-4'); 6.75 (d, *J =* 8.5 Hz, 2H, H-2", H-6"); 7.27 (d, *J=* 8 Hz, 1H, H-3'); 7.42 (d, *J=* 8.5 Hz, 2H, H-3", H-5").
- HRMS ESI (+) m/z: calculated mass for C₂₃H₂₂N₅O₄ 432.1594, found 432.1599.

### Example 11. Preparation of 4,4'-((1H-indol-3-yl)methylene)-bis(3-methyl-1H-pyrazol-5-ole) (11)

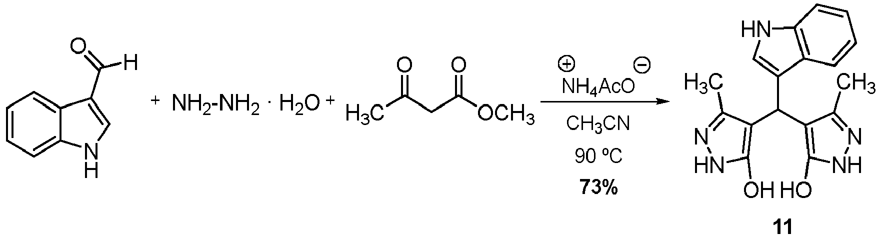

A 50 mL round-bottomed flask with a magnetic stirring bar and cooled down to 0 ± 10 °C with an ice-bath, was charged with hydrazine (d = 1.03 g/mL, 0.43 mL, 5.52 mmol, 2 eq), methyl acetoacetate (0.60 mL, 5.52 mmol, 2 eq), the initial aldehyde (0.400 g, 2.76 mmol, 1 eq) and finally with ammonium acetate (0.400 g, 5.52 mmol, 2 eq). All compounds were dissolved in 5 mL of ACN. The resulting mixture was closed with a reflux condenser and the system was heated at 90 ± 10 °C under constant stirring for 24 hours. During the reaction a spontaneous solid was formed, which was filtered under reduced pressure. The obtained solid was dried and ¹H spectrum was carried out. No purification by column chromatography was performed since the product was obtained with enough purity as shown by TLC and ¹H spectrum. An orange solid was obtained.
*Yield:* 73%

### Analytical data

- R_{f}: 0.02 (Hexane/Ethyl Acetate (1:1))
- Melting point: 225-230 °C (Methanol)
- Aspect: orange solid
- ¹H NMR (DMSO-d₆, 400 MHz) δ(ppm), 2.12 (s, 3H, CH₃); 2.23 (s, 3H, CH₃-); 5.01 (s, 1H, CH-); 6.95 (t, *J=* 7.5 Hz, 1H, H-5'); 7.25 (t, *J=* 7.5 Hz, 1H, H-6'); 7.28 (s, 1H, H-2'); 7.53 (d, *J=* 7.5 Hz, 1H, H-7'); 7.89 (bs, 1H, NH); 8.07 (d, *J=* 7,5 Hz, 1H, H-4').

### Example 12. Preparation of 4,4'-(1-(4-fluorophenyl)ethane-1,1-diyl)-bis(3-methyl-1H-pyrazol-5-ole) (12)

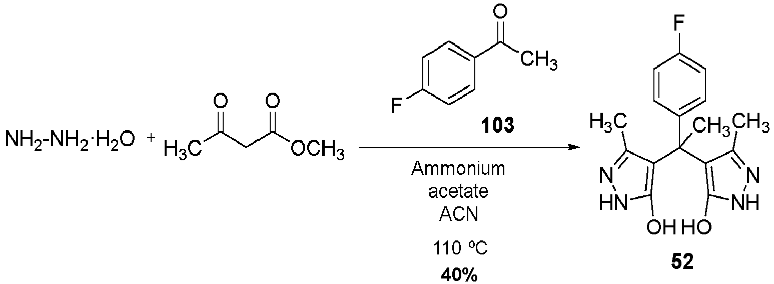

A 50 mL round-bottomed flask equipped with a magnetic stirring bar and cooled down to 0 ± 10 °C with an ice-bath, was charged with hydrazine (d = 1.03 g/mL, 0.16 mL, 3.34 mmol, 2 eq), methyl acetoacetate (0.36 mL, 3.34 mmol, 2 eq), the initial ketone (0.200 g, 1.67 mmol, 1 eq) and finally with ammonium acetate (0.250 g, 3.34 mmol, 2 eq). All components were dissolved in 5 mL of ACN. The resulting mixture was closed with a reflux condenser and the system was heated at 90 ± 10 °C under constant stirring for 24 hours. During the reaction time a spontaneous solid was formed, which was filtered under reduced pressure. The obtained solid was dried and ¹H spectrum was carried out. No purification by column chromatography was performed since the product was obtained with enough purity as shown by TLC and ¹H spectrum. A brown-yellowish solid was obtained.
*Yield:* 40%

### Analytical data

- R_{f}: 0.01 (Hexane/Ethyl Acetate (8:2))
- Melting point: 192-195 °C (Methanol)
- Aspect: brown-yellowish solid
- ¹H NMR (DMSO-d₆, 400 MHz) δ(ppm), 2.20 (s, 3H, CH₃); 2.52 (s, 6H, CH₃ (x2)); 7.11 (t, *J =* 8.5 Hz, 1H, H-3'); 7.31 (t, *J =* 8.5 Hz, 1H, H-5'); 7.33 (t, *J* = 7.7 Hz, 1H, H-2'); 8.02 (t, *J =* 7.7 Hz, 1H, H-6').

**Example 13. KRAS Docking (****Figures 1** **and** **2****).** Before starting the proper docking, K-Ras selected structures were extracted from the trajectory of the MD simulation and water molecules and counter-ions were removed. The docking process was performed with MOE. Two different databases were studied. For conformational analysis the set of ligands were treated in a flexible manner by rotating rotatable bonds. Since all the analyzed molecules were rather small, the systematic method was used to search for all the possible structures, setting a conformational limit of 300 structures. In a first step, receptor structures remained fixed. For placement we used Triangle Matcher method and the Affinity dG scoring function was used to assess candidate poses. After performing the docking of all the compounds of the database, those compounds with binding energy higher than zero were removed. In a second step, a refined docking with induced fit, which allows the free movement of the ligand inside the pocket as well as the movement of the lateral chain of the nearby residues to accommodate the ligand, was performed with the 1000 best molecules of the first step.

Binding analysis of the selected compounds. The specificities of the binding of the selected compounds were analyzed by performing cMD simulations of K-Ras with either ligand. The same methodology as before was followed to prepare the system and carry out the minimizations. Afterwards, the binding free energy of the protein with the ligand along the time was analyzed with the MM/PB(GB)SA methodology.

Compound P14 (Figure 1) was selected to biochemically study its interaction with KRAS, and to analyze putative effects on treated cancer cells signaling and viability. Some derivatives of P14 were also included in the investigation (P14A to P14D; Fig. 2). For compound P14 and the most promising of its derivatives, P14B, a cMD of 100ns length was done to analyze their interaction with KRAS. Moreover, the binding modes at the end of the molecular dynamics are shown in Figure 1C and 1D for compounds P14 and P14B, respectively. Both compounds remained at the binding site described for the KRAS/CaM interaction although their binding mode differed.

The compounds that we identified are activators of RAS signaling instead of inhibitors. Although they all interact with the same KRAS surface, the specific amino acids of KRAS interacting with the compounds may differ, and hence, the differences observed on KRAS signaling. For instance, in contrast to KAL-21404358, our two compounds, P14 and P14B, interact with Glu168, and Lys169 of KRAS.

**Example 14.** Methods. The following methods were used in Examples 15-17.

### Cell lines and culture conditions

DLD-1 (KRASWT/G13D) (clone V15, #HD PAR-086) colorectal adenocarcinoma cell line and DLD-1 knockout of mutant KRAS allele, DLD-1 KO (KRASWT/-) (clone DWT7, #HD105-002), were obtained from Horizon Discovery Ltd. (Cambridge, UK). hTERT-RPE (KRASWT/WT) immortalized retinal pigment epithelial human cell line was obtained from the American Tissue and Cell Collection (ATCC). DLD-1 KO cells stably expressing HA-KRAS G12V were previously generated in our laboratory (Cabot et al., 2021). All cells were grown in DMEM-HAM s F12 (1:1) supplemented with 10% foetal bovine serum (Biological Industries, Israel), penicillin, streptomycin, and nonessential amino acids. Cells were tested once per month for mycoplasma contamination.

### Drug treatment and EGF-dependent signalling activation

Cells were seeded in a media containing 10% FBS for 24 hours and then they were serum starved (0,1 % FBS) for the next 24 hours. Afterwards, they were incubated with the different compounds during the times specified in each figure. When indicated, also in order to activate downstream KRAS cell signalling, treatment for 10 minutes with EGF (50 ng/mL)

(Sigma-Aldrich) was performed.

### Western blot (WB) and antibodies

Proteins were resolved by SDS-PAGE, transferred onto PVDF membranes (Immobilon-P, Millipore) and Western blot was performed as previously described (Cabot et al, Oncogene 2021). The following primary antibodies were used: anti- c-RAF (BD Transduction 610151, 1:500); anti-phospho-c-RAF S338 (Cell Signaling 9427, 1:500); anti-AKT (Cell Signaling 9272, 1:1000); anti-phospho-AKT T308 (Cell Signaling 4056, 1:1000); anti-phospho-MEK1/2 S221 (Cell Signalling 2338, 1:000); anti-p44/42 MAPK (ERK1/2) (Cell Signaling 9102, 1:2000); anti-phospho-p44/42 MAPK(ERK1/2) T202/Y204 (Cell Signaling 4370, 1:2000); anti-HA (Sigma-Aldrich H6908, 1:1000); anticleaved-caspase-3 (Asp175) (Cell Signaling 9661, 1:1000); HRP-coupled secondary antibodies used: goat anti-rabbit (BioRad 170-6515, 1:3000) or goat anti-mouse (BioRad 170 6516, 1:3000).

For analysis of RAS signalling, cells were lysed in a buffer containing 67 mM Tris-HCl pH 6.8 and 2% SDS and then the samples were heated at 97 °C for 15 minutes. Protein concentration of the lysates was assessed using the Lowry method. An aliquot of 15 g of protein per sample was loaded onto the gels.

### Surface Plasmon Resonance Analysis

Surface Plasmon Resonance Analysis was performed by using BIAcore T200 equipment. GST-KRAS (1-166) and GST were covalently immobilized on two of the channels of CM5 Series S Chip following manufacturers instruction. KRAS was loaded with GTP by injecting 1mM of GTP 10µl/min for 30 min in exchange buffer ( 20 mM Tris-HCl pH 7.5; 50mM NaCl, 5% glycerol, 0.1% Triton X-100, 1 mM DTT) with 10 mM EDTA at 30°C and loading was blocked by injecting exchange buffer with 15 mM MgCl2 at 10ul/min for 5 min. P14B was injected at 12,5 µM, 25 µM, 38.5 µM, 50 µM, 75 µM, 100 µM and 150 µM in running buffer (150 mM NaCl, 50 mM Tris-HCl pH7.5, 2 mM MgCl2, 0.1% Triton and 5%DMSO °C. Dissociation was allowed for 10 min in the same buffer. All runs were done by duplicate. Nonspecific binding was subtracted by using two linked channels (GST-KRAS minus GST). Diverse solutions (from 3% to 8%) with DMSO were also prepared to analyse its effect in the RUs, and a solvent correction was performed to reduce the error associated with the injection of the sample.

### Co-Immunoprecipitation

DLD-1 KO cells stably expressing HA-KRAS G12V were serum starved for the next 24 hours before treatment with the compounds for the times indicated. Next, an IP with anti-HA antibody crosslinked to agarose beads (clone HA-7, Sigma-Aldrich A20956) was performed as previously described (Cabot et al, Oncogene 2021).

### Calmodulin (CaM)-pull down

5 g of recombinant GST-KRAS (1-166) (GTP loaded) and 12,5 I of CaM-sepharose beads (Cytiva; Merck) (previously washed with pull-down buffer (PDB) containing 50 mM Tris-HCl, pH 7.5, 150 mM NaCl, 0.1% (v/v) Triton X-100) were incubated in the presence of 1 mM CaCl2 or 5 mM EGTA (in a total volume of 100 I with PDB) for 60 minutes at room temperature. The unbound fraction was collected by centrifugation, and the bound fraction was washed four times with PDB with either CaCl2 or EGTA. The entire bound fraction was analysed by WB.

### Purification of GST-KRAS (1-166)

GST-KRAS (1-166) fusion protein was expressed in Escherichia coli BCl21 and then purified and loaded with the specific nucleotide as previously described (Villalonga et al., 2001).

### Cell viability assay

10,000 cells in 50 L of 10% FBS-containing medium, were cultured for 24 hours and then treated with the compounds (50 L final volume) for 48h hours in each well of a 96-well plate (100 µL final volume). MTS viability assay (CellTiter 96^{®} Aqueous One Solution Cell Proliferation Assay, Promega G3580) was assessed following the company conditions. The absorbance of each well was measured with a multimode plate reader (Spark, Tecan) at 490 nm. The percentage of cell viability was calculated by dividing the absorbance of each well by the average absorbance of the control wells

### 3-Dimensional (3D) cell culture 608

3D on-top Matrigel assay was performed as described in (Cabot et al., 2021).

### Statistical analysis

Statistical analyses were performed with GraphPad Prism 8.1. Data shown represent the mean ± SEM or SD (as indicated in figure legends) of three or four independent experiments. Significant differences were assessed using one-way ANOVA with multiple comparisons tests or t-Student test and considered when P < 0.05.

**Example 15. Compounds of the invention induce higher activation of Ras effectors ERK and AKT than control (****Figure 3****).**

We first analyzed the effect of P14 on the activation of downstream RAS signaling pathways (RAF/MEK/ERK and PI3K/AKT) in DLD-1 cells (CRC cells carrying one oncogenic KRAS allele). To this end, DLD-1 serum-starved cells (0.1% FCS) were treated with P14 (100 µM) at different times, and activation of AKT and ERK was evaluated by Western blot. The data showed that P14 significantly increased P-ERK and P-AKT at 3 and 6 hours of cell treatment (Figure 3A). As a 3-hour treatment was associated with major effects, we chose this duration for the subsequent experiments with the other compounds. Next, DLD-1 serum-starved cells were treated with P14A-P14C at 100 µM for 3 hours and activation of AKT and ERK was assessed as above. The results showed that P14, P14B, and P14C activated AKT and ERK kinases, with P14 and P14B being the most efficient. Since P14B was the compound with the greatest capacity to activate Ras signaling, even at levels comparable with those reached with 10% FBS (at 30 min), we selected it for further study. The kinetics of RAS downstream signaling was analyzed in P14B-treated cells compared to 10% FBS serum. As shown in Figure 2C, kinetics of AKT, MEK and ERK phosphorylation differed. ERK and MEK activation was clearly delayed in P14B-treated cells compared with 10%-treated ones.

Additionally, and especially in the case of ERK, its phosphorylation was maintained for a longer time. Thus P14B, a small compound that was shown to interact with the α4-α5 surface of KRAS in silico, was able to induce a sustained increase in KRAS signaling. Treatment of DLD-1 cells increased endogenous downstream RAS signaling with different compounds of the invention. (B) shows Western blot of P-AKT and P-ERK expression of cells treated with compounds of the invention. Specifically, DLD-1 starved cells were incubated for 3 hours with P14B, P14C or P14D, or for 30 minutes with 10% FBS or 0.1 % FBS, or for 10 minutes with 50 ng/mL of EGF. The levels of activation and the total levels of RAF, MERK, ERK and AKT were analyzed by Western blot with specific antibodies against the active phosphorylated forms of these kinases or against the total forms of these proteins, respectively. (D) and (E) show quantification of P14, P14A, P14B, or P14C of the Western blot shown in (B).

Since our aim was to analyze the effect on signaling pathways activated by oncogenic KRAS, we used DLD-1 cells, which are CRC cancer cells harboring one oncogenic KRAS allele. Even in the presence of this oncogenic allele, the addition of FBS to serum-starved cells, induced an increase in P-MEK, P-ERK and P-AKT levels. That means that additional signals induced by growth factors allow full activation of the signal transduction pathways. Remarkably, addition of P14B alone was also enough to achieve this activation. Furthermore, ERK phosphorylation was more sustained in P14B-treated cells than in FBS treated cells, suggesting that some of the negative feedback pathways to deactivate ERK (Lake et al., 2016) were provably not induced by P14B.

Conclusion: All of P14, P14A, P14B, and P14C increase AKT and/or ERK signaling. P14 A and B induce the highest activation of both Ras effectors: ERK and AKT.

**Example 16. Direct interaction between KRAS and P14B** (Figure 4). To confirm the direct interaction between KRAS and P14B, surface plasmon resonance analysis was performed. Purified GST-KRAS (aminoacids 1 to 166) was immobilized and then GTP loaded. After using different concentrations of P14B as the analyte, an affinity constant (KD) of 32.8 µM of P14B for GTP-loaded KRAS was determined (Figure 3A). Given that P14B is able to bind to oncogenic KRAS (Figure 3) most likely via the same surface that KRAS uses to interact with CaM, we analyzed the possibility that P14B can displace CaM from oncogenic KRAS. To this end, we performed an in vitro competence assay that consisted of pulling-down recombinant GTP-loaded GST-KRAS-G12V (1-166) with CaM-sepharose beads followed by incubation with increasing concentrations of P14B.

Figure 3B shows that Ca+2-dependent GST-KRAS-G12V binding to CaM was reduced in the presence of P14B.

**Example 17. P14B favors the interaction of oncogenic KRAS with BRAF and phosphorylated C- 239-RAF.** (Figure 5). We assessed the possibility that P14B favors RAS-effector binding and consequently increases downstream RAS signaling. To this end, co-immunoprecipitation experiments were carried out to analyze the interaction of KRAS with different RAF family members upon P14B treatment. The interaction was analyzed after 10 min of P14B treatment since P-C-RAF and P-MEK were observed after 15 min (Figure 3C). DLD-1 cells stably expressing HA-KRAS-G12V were serum-starved and then treated for 10 min with either EGF or P14B. P14B induced phosphorylation of AKT and ERK in these cells (Figure 5A). As expected, the co-immunoprecipitation analysis showed that C-RAF interacted with oncogenic KRAS under serum-starvation conditions, but this immunoprecipitated C-RAF was phosphorylated only upon EGF treatment (Figure 5B). Interestingly, treatment with P14B increased the levels of P-C-RAF co-immunoprecipitated with KRAS to levels similar to those reached by EGF treatment (Figure 5B). In contrast, BRAF did not bind to oncogenic KRAS under serum-starved conditions, and upon EGF stimulation the interaction of these two proteins was observed. Interestingly, P14B treatment induced the same effect as EGF and an increase in the KRAS-BRAF interaction was observed with respect to non-treated cells (Figure 5B). No differences were detected regarding KRAS-ARAF binding when cells were treated with P14B. The assays presented here prove that the treatment of CRC cells with P14B intensifies oncogenic KRAS interaction with P-C-RAF (S338) and BRAF, in agreement with the positive impact of this compound on downstream RAS signaling.

**Example 18.** Viability of CRC cells expressing oncogenic KRAS is impaired by treatment with P14B, but not that of normal cells (Figure 6). Since both inhibition and sustained activation of RAS signaling are related to a decrease in cell survival, we analyzed the viability of the CRC cell lines DLD-1 (expressing oncogenic KRAS) and DLD-1-KO (oncogenic KRAS allele deleted), and the non-transformed cell line hTERT-RPE. Doseresponse experiments (from 0 to 100 µM) at 24 hours showed interesting results: P14B significantly reduced the cell viability of DLD-1, specifically at 75 µM and 100 µM. At the IC50 of approximately 80 µM for DLD-1 cells, the viability of DLD-1-KO and of non-transformed cells was not affected (Figure 5A). Interestingly, no significant differences in cell viability were observed between DLD-1-KO and hTERT-RPE. The effect of P14B in DLD-1 cells cultured in 3D conditions was also determined. When P14B was added when seeding the cells, a drastic reduction in the capacity to form colonies in Matrigel was observed even at 10 µM. When P14B was added 24 h after seeding (when colonies were already formed), a clear reduction in the size of the colonies was observed at 40 µM (Figure 5B). Finally, to determine whether cells were dying by apoptosis, the three cell lines were treated with P14B for 34 h or 48 h, and the protein expression of cleaved caspase-3 was analyzed by WBAs shown in Figure 5C, cleaved caspase-3 was only detected in DLD-1-treated cells being undetectable in the samples of DLD-1-KO and hTERT-RPE. Consequently, we conclude that P14B selectively reduces the viability of CRC cells expressing oncogenic KRAS, causing minor effects if these cells have been knocked out for oncogenic KRAS or in non-transformed cells. Thereby, P14B might be considered as an inducer of apoptosis in CRC with oncogenic KRAS.

**Example 19. Comparison of Ras signaling between DLD-1, DLD-1-KO and hTERT-RPE cells** (Figure 7). Comparison of Ras signaling between DLD-1, DLD-1-KO and hTERT-RPE cells treated with P14B since important differences were observed between the viability of cells treated with P14B depending on their expression or lack of expression of oncogenic KRAS, we further studied whether these discrepancies were reflected also in KRAS downstream signaling. Serum-starved cells (0.1% FCS) were treated with P14B (100 µM) and activation of AKT and ERK was evaluated by WB. Results show that while AKT and ERK phosphoryation dramatically increased after the P14B treatment of DLD-1 cells, reaching levels comparable with those reached with 10% FBS, no significant increase was observed in hTERT-RPE cells treated with P14B. Regarding DLD-1-KO, AKT and ERK activation by P14B was also observed, but the levels of ERK phosphorylation were significantly lower than the ones reached upon 10% FBS treatment (Figure 7). Thus, cell death observed in the different cell lines positively correlated with an increase in ERK activation.

**Example 20. Study the effect of Erlotinib and P14B in cell viability of DLD-1 (Colorectal cancer with one oncogenic KRAS allele) cell line treated with different concentrations of the indicated compounds** (Figure 8).

5000 cells in 50 µL of 10% FBS-containing medium, were cultured for 24 hours and then treated with the compounds (50 µL final volume) for 48h hours in each well of a 96-well plate (100 µL final volume). Each point corresponds to n=6. The concentration (µM) indicated in the graph is the one in the final 100 µL volume). **** p<0.0001 (t-student test). In DLD1 cells, the maxim cell viability reduction induced by P14B is significantly higher than the one induced by Erlotinib.

### CITATION LIST

Cabot, D., Brun, S., Paco, N., Ginesta, M.M., Gendrau-Sanclemente, N., Abuasaker, B., Ruiz-Fariña, T., Barceló, C., Cuatrecasas, M., Bosch, M., et al. (2021). KRAS phosphorylation regulates cell polarization and tumorigenic properties in colorectal cancer. Oncogene 40, 5730 5740.
Canon, J., Rex, K., Saiki, A.Y., Mohr, C., Cooke, K., Bagal, D., Gaida, K., Holt, T., Knutson, C.G., Koppada, N., et al. (2019). The clinical KRAS(G12C) inhibitor AMG 510 drives anti-tumour immunity. Nature 575, 217 223.
Cox, A.D., Fesik, S.W., Kimmelman, A.C., Luo, J., and Der, C.J. (2014). Drugging the undruggable RAS: Mission Possible? Nat. Rev. Drug Discov. 13, 828 851.
Hallin, J., Engstrom, L.D., Hargi, L., Calinisan, A., Aranda, R., Briere, D.M., Sudhakar, N., Bowcut, V., Baer, B.R., Ballard, J.A., et al. (2020). The KRASG12C inhibitor MRTX849 provides insight toward therapeutic susceptibility of KRAS-mutant cancers in mouse models and patients. Cancer Discov. 10, 54 71.
Karnoub, A.E., and Weinberg, R.A. (2008). Ras oncogenes: Split personalities. Nat. Rev. Mol. Cell Biol. 9, 517 531.
Lake, D., Corrêa, S.A.L., and Müller, J. (2016). Negative feedback regulation of the ERK1/2 MAPK pathway. Cell. Mol. Life Sci. 73, 4397 4413.
Malumbres, M., and Barbacid, M. (2003). RAS oncogenes: the first 30 years. Nat. Rev. Cancer 3, 459 465.
McCormick, F. (2019). Progress in targeting RAS with small molecule drugs. Biochem. J. 476, 365 374.
Nobuo, T., Tom, R., and Eiichi, O. (1982). Nucleotide Sequence of the Oncogene Encoding the p21 Transforming Protein of Kirsten Murine Sarcoma Virus. Science (80). 217, 937 939.
Prior, I.A., Hood, F.E., and Hartley, J.L. (2020). The Frequency of Ras Mutations in Cancer. Cancer Res. 80, 2969 2974. Pylayeva-Gupta, Y., Grabocka, E., and Bar-Sagi, D. (2011). RAS oncogenes: Weaving a tumorigenic web. Nat. Rev. Cancer 11, 761-774. Simanshu, D.K., Nissley, D. V., and McCormick, F. (2017). RAS Proteins and Their Regulators in Human Disease. Cell 170, 17 33.
Lopez-Alcalá C, et al. J Biol Chem. 2008 Apr 18;283(16):10621-31. doi: 10.1074/jbc.M706238200.

## Claims

1. A compound having the general formula I 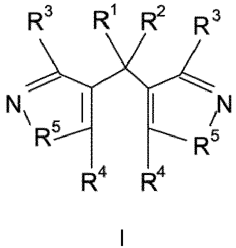 wherein
R' is selected from H, branched, cyclo or linear (C₁-C₆)-alkyl, branched, cyclo or linear (C₁-C₆)-alkyl-OH, branched, cyclo or linear (C₁-C₆)-alkyl-NH₂, branched or linear (C₁-C₆)-alkyl comprising a secondary or tertiary amine or an ether group;
R² is selected from branched, cyclo or linear (C₁-C₆)-alkyl, heterocyclyl, substituted or unsubstituted (C₆-C₁₀)-aryl, or substituted or unsubstituted (C₅-C₈)-cycloalkenyl,
wherein the substituted (C₆-C₁₀)-aryl or substituted (C₅-C₈)-cycloalkenyl is substituted with 1 or 2 groups selected from the groups consisting of
a) OCH₃,
b) halogen,
c) a substituent having formula II 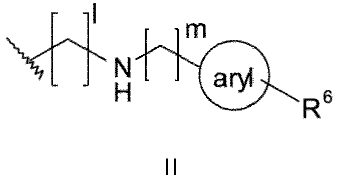 wherein "I" and "m" are independently selected from 0 to 6, the aryl group has 5 to 10 atoms, and wherein R⁶ is H, branched, cyclo or linear (C₁-C₆)-alkyl, or halogen;
d) a substituent having formula III 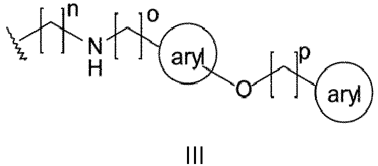 wherein the aryl groups independently have 5 to 10 atoms, and "n", "o" and "p" are independently selected from 0 to 6; and/or
e) a substituent having formula IV 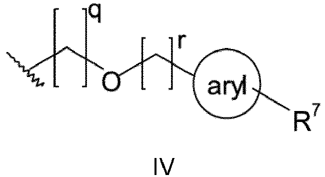 wherein "q" and "r" are independently selected from 0 to 6, the aryl group has 5 to 10 atoms, and wherein R⁷ is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1;
R₃ is independently selected from H, branched, cyclo or linear (C₁-C₆)-alkyl, branched, cyclo or linear (C₁-C₆)-alkyl-OH, CF₃, halogen, NO₂, and CN;
R₄ is independently selected from H, OH, O, NH₂, and NO₂; and
R⁵ is independently selected from N, NH, O, S, branched, cyclo or linear (C₁-C₆)-alkyl, branched or linear (C₁-C₆)-alkyl comprising a secondary or tertiary amine, an ether group or a sulfide group, N=N, and CH=N.

2. The compound of claim 1, wherein R¹ is selected from H and CH₃.

3. The compound of claim 1 or 2, wherein the heterocyclyl is selected from indolyl, pyrrolyl, furanyl, thiophenyl, pyridyl, pyrimidyl, pyridazyl, pyrazinyl, quinolinyl, isoquinolinyl, acridinyl, 1,2-methylenedioxyphenyl or 1,2-ethylenedioxyphenyl.

4. The compound of any one of claims 1 to 3, wherein the substituted or unsubstituted (C₆-C₁₀)-aryl is selected from substituted or unsubstituted phenyl, substituted or unsubstituted tolyl, substituted or unsubstituted xylyl, and substituted or unsubstituted naphthyl, preferably substituted phenyl.

5. The compound of any one of claims 1 to 4, wherein R² is indol-2- or 3-yl, pyrrol-3-yl, substituted phenyl, 1,2-methylenedioxyphenyl, 1,2-ethylenedioxyphenyl, 2- or 3-pyrrolyl, 2- or 3-furanyl, 2- or-thiophenyl, 2-, 3- or 4-pyridyl, 2- 4 or 6-pyrimidyl, quinolinyl, or isoquinolinyl.

6. The compound of any one of claims 1 to 5, wherein the aryl group has 5 to 10 atoms in formulae (II), (III) and/or (IV) is selected from phenyl, pyrrolyl, furanyl, thiophenyl, pyridyl, pyrimidyl, quinolinyl and/or isoquinolinyl, preferably phenyl.

7. The compound of any one of claims 1-6, wherein the compound is selected from

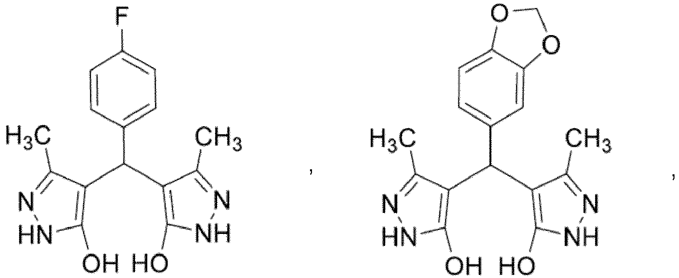

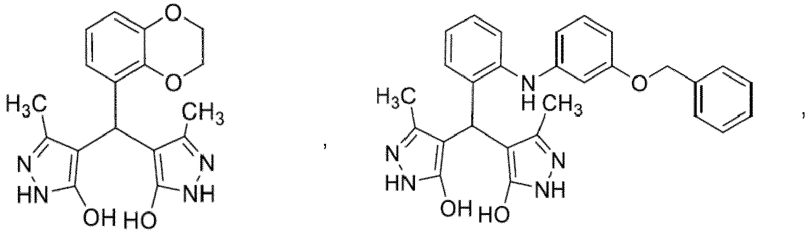

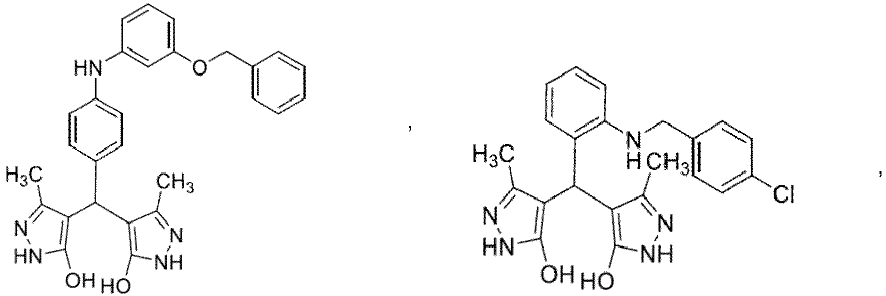

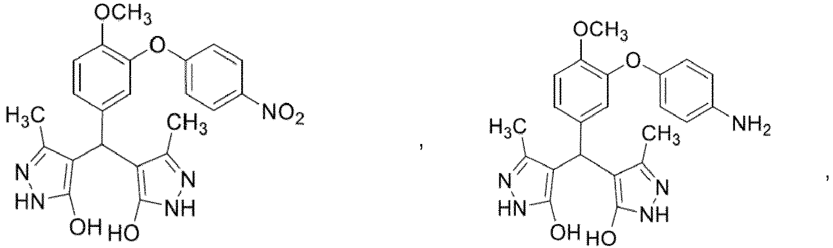

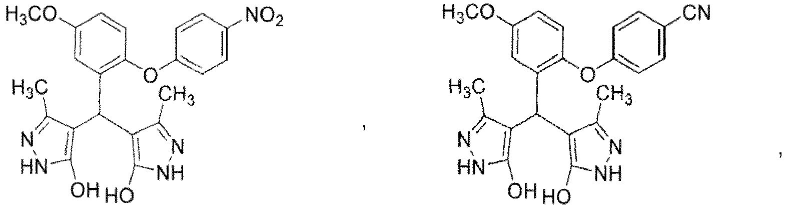

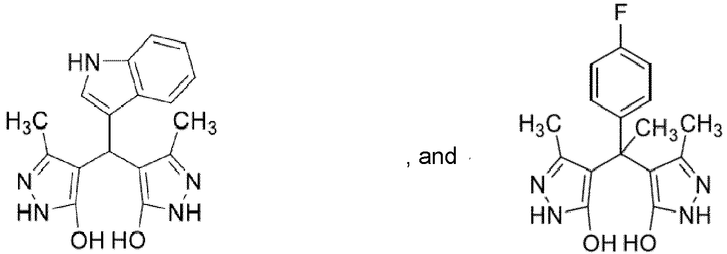

8. An intermediate compound in the synthesis of compounds of any one of claims 1 to 7, wherein the intermediate compound is selected from
a) branched, cyclo or linear (C₁-C₆)-alkyl substituted with CHO;
b) heterocycle substituted with CHO;
c) (C₅-C₈)-cycloalkenyl substituted with CHO;
d) (C₆-C₁₀)-aryl substituted with CHO;
e) (C₅-C₈)-cycloalkenyl, or the (C₆-C₁₀)-aryl are substituted with CHO and with 1 or 2 groups selected from
i) OCH₃;
ii) halogen;
iii) a substituent having formula II 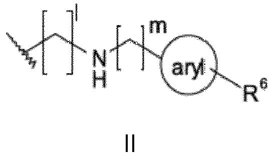 wherein "I" and "m" are independently selected from 0 to 6, the aryl group has 5 to 10 atoms, and wherein R⁶ is H, branched, cyclo or linear (C₁-C₆)-alkyl, or halogen;
iv) a substituent having formula III 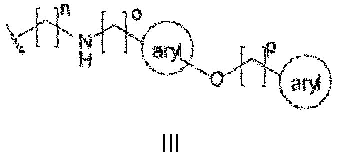 wherein the aryl groups independently have 5 to 10 atoms, and "n", "o" and "p" are independently selected from 0 to 6; and/or
v) a substituent having formula IV 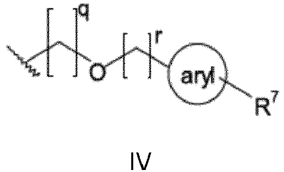 wherein "q" and "r" are independently selected from 0 to 6, the aryl group has 5 to 10 atoms, and wherein R⁷ is selected from halogen, NO₂, NH₂, CN, CF₃, and branched, cyclo or linear (C₁-C₆)-alkyl; wherein R⁷ cannot be Cl, when the substituent of formula IV is the only substituent of the (C₆-C₁₀)-aryl and "q" is 0 and "r" is 1.

9. The compound as defined in any one of claims 1-7 or a salt or solvate thereof or a compound of formula 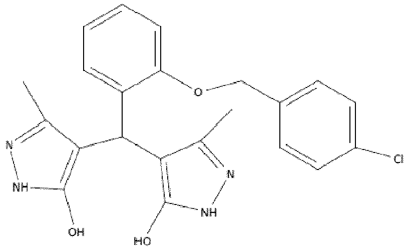 or a salt or solvate thereof for use as a medicament.

10. The compound as defined in any one of claims 1-7 or a salt or solvate thereof or a compound of formula 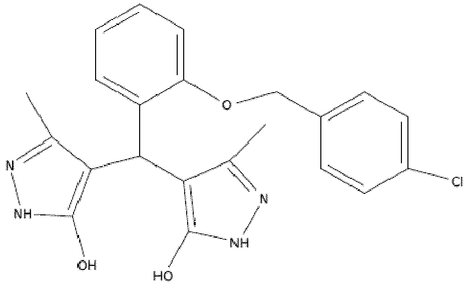 or a salt or solvate thereof
for use in treating or preventing pancreatic cancer, lung cancer, colorectal cancer, thyroid cancer, testicle cancer, melanoma, bladder cancer, liver cancer, kidney cancer, myelodysplastic syndrome, or leukemia.

11. A pharmaceutical composition comprising the compound as defined in any one of claims 1-7 or a pharmaceutically acceptable salt or solvate thereof or a compound of formula 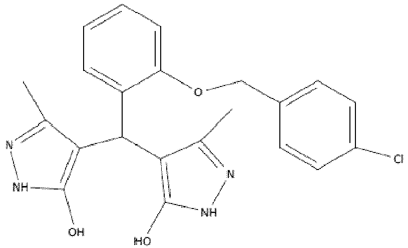 or salt or solvate thereof.

12. The pharmaceutical composition of claim 11 for use as a medicament.

13. The pharmaceutical composition of claim 11 or 12 for use in treating pancreatic cancer, lung cancer, colorectal cancer, thyroid cancer, testicle cancer, melanoma, bladder cancer, liver cancer, kidney cancer, myelodysplastic syndrome, or leukemia.

14. A method for the preparation of a compound as defined in any one of claims 1-33 or the pharmaceutical composition of claim 55, the process comprising contacting 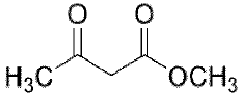 NH₂-NH₂·H₂O with and with one intermediate compound, wherein the intermediate compound is defined in claim 8.

15. A kit or package comprising the compound as defined in any one of claims 1-7, or the pharmaceutical composition of claim 11.
